# EUROPEAN PATENT SPECIFICATION

(11) **EP 4 026 515 B1**
(45) Date of publication and mention of the grant of the patent: **25.12.2024**
(21) Application number: 20868715.2
(22) Date of filing: 21.08.2020
(51) Int. Cl.: A61C 13/00, A61C 9/00, A61C 13/097, A61C 13/34, A61B 5/00, A61C 8/00, A61C 5/77

(54) **METHOD FOR MANUFACTURING IMPLANT SHAPE FOR MINIMIZING THE STRESS APPLIED TO IMPLANT BY USING COMPUTER**
VERFAHREN ZUM HERSTELLEN EINER IMPLANTATFORM ZUR MINIMIERUNG DER DURCH EINEN COMPUTER AUF DAS IMPLANTAT AUSGEÜBTEN SPANNUNG
PROCÉDÉ DE FABRICATION D'UNE FORME D'IMPLANT VISANT À MINIMISER LA CONTRAINTE APPLIQUÉE À UN IMPLANT EN UTILISANT UN ORDINATEUR

(30) Priority: 27.09.2019 KR 20190119378; 18.08.2020 KR 20200103331
(43) Date of publication of application: 13.07.2022
(73) Proprietor: Pusan National University Industry-University Cooperation Foundation, Busan 46241 (KR); University-Industry Foundation, Yonsei University, Seoul 03722 (KR)
(72) Inventor: LEE, Hyeon Jong, Busan 48050 (KR); HWANG, Jae Joon, Busan 46294 (KR)
(74) Representative: BCKIP Part mbB
(86) International application number: PCT/KR2020/011198
(87) International publication number: WO 2021/060713

(56) References cited:
- JP-B1- S5 220 626
- KR-A- 20160 083 787
- KR-A- 20180 060 502
- KR-A- 20190 065 590
- KR-B1- 100 971 762
- US-A1- 2009 068 617
- US-A1- 2010 054 558
- US-A1- 2017 258 561
- US-A1- 2019 147 666

## Description

### Technical Field of the Invention

The present invention relates to a method of forming, by utilizing a computer, an implant shape for minimizing stress to be applied to an implant. More particularly, the present invention serves the purpose of adjusting force acting on an occlusal adjustment surface of an implant crown in such a manner that a direction of the force is closest to an implanted direction of an implant and of minimizing stress applied to the implant.

### Background Art

In the Republic of Korea, implants were introduced in the 1980s, and dental-implant insurance benefit has been provided to persons over the age of 65 since 2016. In recent years, implant surgery has become one of popular dental prosthetic treatments. Adverse effects, such as alveolar bone loss due to implant unscrewing, fatigue failure, and micro-fluctuation, increase with an increase in the number of times that implant surgery is performed.

In order to reduce these adverse events, technologies for treating a surface of an implant itself to increase a speed at which the implant and bone cells are fused together and to minimize a bone loss, technologies for increasing resistance to mechanical stress through an improved strength and physical property of an implant material, and the like have been disclosed. However, there are limitations that follows. In most cases where it is necessary to implant the implant, a thick implant is difficult to implant because an amount of bone is insufficient. Furthermore, it is not easy to develop a material that is applicable to clinical surgery and has improved strength and physical properties.

The result of prior research on the factors that have an influence on stress applied to the implant shows that stress applied to the implant along a direction of an external force applied to the implant under the same condition varies by up to 3.5 times as high.

Currently, for implant restoration, a tooth shape is designed and formed using a computer software program, such as Computer-Aided Designing & Manufacturing (CAD-CAM).

In the CAD program, a crown library is automatically set for a missing tooth. However, there is a problem in that many amendments need to be made on the crown library because the crown library is not customized for an individual patient that brings an existing crown shape.

In the CAD program, a virtual occluder is present. However, manual operation of the occluder for occlusal adjustment makes the occluder difficult to operate actually while a dental technician is busy in constructing a custom-made restorative and dental appliance. Analysis of a crown designed with the CAD program can visualize the direction in which an average of external forces is exerted.

With reference to this visualized direction, an implant crown can be designed in such a manner that the direction of the external force is toward the center of the tooth root, that is, the center of an implant. Thus, a numerical value of stress can be more minimized than when the external force is exerted toward the outside of the implant.

The use of the CAD program makes it possible to visualize the direction in which the external force is applied to the implant, but does not take stress into consideration because the implant design is mostly made by the dental technician. In addition, although the implant is designed taking stress into consideration, a process of redesigning the implant clown after checking the direction in which the external force is exerted on the implant needs to be reiterated until the direction in which the external force is exerted on the implant is toward the axis of the implant. This process is time-consuming.

Document US 2017 / 258 561 A1 discloses a method for digitally designing a dental restoration, wherein a 3D representation of at least a part of the upper or lower jaw is obtained. The 3D representation represents at least a target site for placing the final restoration and at least one antagonist tooth opposing the target site. In addition, a digital anatomy design of the restoration is provided based at least on a dynamic occlusion and a relative offset of the planned restoration position.

### Summary of the Invention

### Technical Problem

An objective of the present invention is to minimize an angle that an external force vector applied by an antagonist tooth to all local portions makes to an implant after extracting an occlusal adjustment surface between an arbitrarily selected crown on a position of a missing tooth and the antagonist tooth, by utilizing a computer program, and thus to minimize stress occurrence.

The present invention is not limited to the above-mentioned objectives. From the following description, other objectives that are not mentioned will be clearly understood.

### Technical Solution

To this end, the present invention provides a method of forming, by a utilizing a computer, an implant shape for minimizing stress to be applied to an implant in accordance with claim 1. There is provided a method of forming, by utilizing a computer, an implant shape for minimizing stress to be applied to an implant, the method including: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle.

According to another aspect of the present invention, there is provided a method of forming, by utilizing a computer, an implant shape for minimizing stress to be applied to an implant, the method including: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle, wherein in a case where the value of the single vector derived in the vector derivation step S8 is such that the angle R with respect to the axis of the virtual implant 100 falls outside a range from 0 degrees to 10 degrees, the occlusal adjustment point 201b of the second temporary crown 200b is redetermined on the basis of an input from a user, and the steps S6 and S7 are reiterated.

According to still another aspect of the present invention, there is provided a method of forming, by utilizing a computer, an implant shape for minimizing stress to be applied to an implant, the method including: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle, wherein a crown shape of the first temporary crown 200a in the first temporary crown arrangement step S4 is determined using positional information of a missing tooth, and the second temporary crown 200b in the second temporary crown substitution step S5 is provided in such a manner that a size of the second temporary crown 200b is adjusted considering a relationship between each of the adjacent teeth 12 and 13 positioned to the left and right, respectively, of the missing tooth and the antagonist tooth 21 engaged with the missing tooth.

According to still another aspect of the present invention, there is provided a method of forming, by utilizing a computer, an implant shape for minimizing stress to be applied to an implant, the method including: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle, wherein a crown shape of the first temporary crown 200a in the first temporary crown arrangement step S4 is determined using positional information of a missing tooth, the second temporary crown 200b in the second temporary crown substitution step S5 is provided in such a manner that a size of the second temporary crown 200b is adjusted considering a relationship between each of the adjacent teeth 12 and 13 positioned to the left and right, respectively, of the missing tooth and the antagonist tooth 21 engaged with the missing tooth, and wherein a crown shape of the first temporary crown 200a in the first temporary crown arrangement step S4 is determined using positional information of a missing tooth, and the second temporary crown 200b in the second temporary crown substitution step S5 is provided in such a manner that a size of the second temporary crown 200b is adjusted considering a relationship between each of the adjacent teeth 12 and 13 positioned to the left and right, respectively, of the missing tooth and the antagonist tooth 21 engaged with the missing tooth.

According to still another aspect of the present invention, there is provided a method of forming, by utilizing a computer, an implant shape for minimizing stress to be applied to an implant, the method including: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle, wherein three to eight occlusal adjustment points 201b in the occlusal adjustment point extraction step S6 are extracted.

In an embodiment not covered by the present invention, there is provided a method of recommending an optimal occlusal adjustment region for an implant by utilizing a computer, the method including: a step of generating a three dimensional model for each of a treatment-target region including a missing-tooth portion and an antagonist tooth region facing the treatment-target region from scan data generated by scanning a patient's oral cavity using an intraoral scanner; a step of arranging a virtual implant abutment in a missing-tooth portion of the three-dimensional model for the treatment-target region; a step of selecting any one tooth model from among a plurally of tooth models as prestored three-dimensional models and setting the selected one tooth model to be a temporary crown; a step of adjusting a size of the temporary crown according to shapes of adjacent teeth adjacent to the missing-tooth portion in the three-dimensional model for the treatment-target region and to a shape of an antagonist tooth corresponding to the missing-tooth portion in the three-dimensional model for the antagonist tooth region and arranging the temporary crown on the top of the virtual implant abutment; a step of generating an optimal occlusal adjustment region where a direction of a composite vector based on orthogonal vectors applied from the antagonist tooth to the temporary crown is toward a lower cross-sectional region of the virtual implant abutment, from the three-dimensional model for the antagonist tooth on the basis of at least two regions where a surface of the temporary crown and a surface of the antagonist tooth are brought into contact with each other, in a case where the temporary crown arranged on the top of the virtual implant abutment and the antagonist tooth are aligned in such a manner as to be engaged with each other; a step of generating an optimal occlusal adjustment region image for displaying the optimal occlusal adjustment region on the three-dimensional model for the antagonist tooth and an antagonist tooth region image for displaying the three-dimensional model for the antagonist tooth region; and a step of displaying the optimal occlusal adjustment region image on the antagonist tooth region image in an overlapping manner.

In another embodiment not covered by the present, there is provided an apparatus for recommending an optimal occlusal adjustment region for an implant by utilizing a computer, the apparatus including: a scan data input unit configured in such a manner that scan data generated by scanning a patient's oral cavity using an intraoral scanner is input from the outside into the scan data input unit; a three-dimensional model generation unit configured to generate a three-dimensional model for each of a treatment-target region including a mission-tooth portion and an antagonist tooth region facing the treatment-target region from the scan data; a first arrangement unit configured to arrange a virtual implant abutment in the missing-tooth portion of the three-dimensional model for the treatment-target model; a temporary crown setting unit configured to select any one tooth model from among a plurality of tooth models as prestored three-dimensional models and setting the selected one tooth model as a temporary crown; a second arrangement unit configured to adjust a size of the temporary crown according to shapes of adjacent teeth adjacent to the missing-tooth portion in the three-dimensional model for the treatment-target region and to a shape of an antagonist tooth corresponding to the missing-tooth portion in the three-dimensional model for the antagonist tooth region and arranging the temporary crown on the top of the virtual implant abutment; an optimal occlusal adjustment region generation unit configured to generate an optimal occlusal adjustment region where a direction of a composite vector based on orthogonal vectors applied from the antagonist tooth to the temporary crown is toward a lower cross-sectional region of the virtual implant abutment, from the three-dimensional model for the antagonist tooth on the basis of at least two regions where a surface of the temporary crown and a surface of the antagonist tooth are brought into contact with each other, in a case where the temporary crown and the antagonist tooth are aligned in such a manner as to be engaged with each other on the top of the virtual implant abutment; an image generation unit configured to generate an optimal occlusal adjustment region image for displaying the optimal occlusal adjustment region on the three-dimensional model for the antagonist tooth and an antagonist tooth region image for displaying the three-dimensional model for the antagonist tooth region; and a display unit configured to display the optimal occlusal adjustment region image on the antagonist tooth region image in an overlapping manner.

### Advantageous Effects

According to the present invention, by utilizing a computer program, an occlusal adjustment surface between an arbitrarily selected crown on a position of a missing tooth and an antagonist tooth is extracted, and then an angle that an external force vector applied by the antagonist tooth to each local portion makes to the implant is minimized. Accordingly, the present invention can achieve the advantageous effect of minimizing stress occurrence.

According to the present invention, stress is applied toward the direction of the center of a virtual implant abutment. Accordingly, the present invention can achieve the advantageous effect of providing on an antagonist tooth an optimal occlusal adjustment region where the stress applied to an implant is minimized.

The present invention can achieve the advantageous effect of designing an implant crown taking the stress into consideration through the use of the optimal occlusal adjustment region without the need for expertise in stress analysis.

The present invention can achieve the advantageous effect of providing a realistic optimal occlusal adjustment region by applying a prestored tooth shape to the tooth-missing portion and generating the optimal occlusal adjustment region.

The present invention is not limited to the above-mentioned advantageous effects. From the claims, other advantageous effects that are not mentioned will be clearly understood.

### Description of Drawings

FIG. 1 is a diagram illustrating the order of steps of a method of forming, by utilizing a computer, an implant shape for minimizing stress to be applied to an implant according to embodiments of the present invention;
FIGS. 2 to 4 are screen views each illustrating a tooth scan model generation step S1;
FIG. 5 is an exemplary screen view illustrating that in a scan file alignment step S2, a treatment-target region and a teeth arrangement facing the treatment-target region are aligned in such a manner as to be engaged with each other using maxillary and mandibular occlusal adjustment information;
FIGS. 6 to 8 are screen views each illustrating a virtual implant arrangement step S3;
FIGS. 9 and 10 are exemplary screen views each illustrating a state where a first temporary crown 200a is arranged on the virtual implant 100 in a first temporary crown arrangement step S4;
FIG. 11 is a screen view illustrating a second temporary crown substitution step S5;
FIG. 12 is an exemplary screen view illustrating an occlusal adjustment point extraction step S6;
FIGS. 13 and 14 are exemplary screen views each illustrating that a vector-value analysis code is executed in order to analyze a vector value in a second temporary crown occlusal adjustment region derivation step S7;
FIG. 15 is a screen view illustrating the second temporary crown occlusal adjustment region derivation step S7;
FIG. 16 is a screen view illustrating an occlusal adjustment region 202b of a second temporary crown 200b derived in the second temporary crown occlusal adjustment region derivation step S7.
FIG. 17 is a screen view illustrating a state where a force applied from every local portion to the antagonist tooth at each point of the occlusal adjustment region 202b illustrated in FIG. 16(a) is expressed on a per-vector basis;
FIGS. 18 and 19 are screen views each illustrating a vector derivation step S8;
FIG. 20 is a screen view illustrating a state where a value of an angle R is decreased in FIG. 18;
FIG. 21 is a screen view illustrating a state where an occlusal adjustment point 201b of the second temporary crown 200b is reselected;
FIG. 22 is a screen view illustrating the value of the angle R resulting from redetermining an occlusal adjustment point in FIG. 21;
FIG. 23 is a block diagram illustrating an apparatus for recommending an optimal occlusal adjustment region for an implant by utilizing a computer according to a second embodiment not covered by the present invention;
FIG. 24 is a block diagram illustrating a method of recommending an optimal occlusal adjustment region for an implant by utilizing a computer according to a third embodiment not covered by the present invention;
FIG. 25 is a flowchart for selecting at least one polygon in a per-candidate occlusal adjustment polygon group basis in FIG. 24;
FIG. 26 is an exemplary screen view illustrating a three-dimensional model for each of a treatment-target region and an antagonist tooth region according to the third embodiment not covered by the present invention;
FIG. 27 is an exemplary screen view illustrating the three-dimensional model for the treatment-target region where a virtual implant abutment is arranged according to the third embodiment not covered by the present invention;
FIG. 28 is an exemplary screen view illustrating the three-dimensional model for the treatment-target region where a temporary crown is arranged according to the third embodiment not covered by the present invention;
FIG. 29 is an exemplary screen view illustrating that the three-dimensional model for the treatment-target region where the temporary crown is arranged and the three-dimensional model for the antagonist tooth region are aligned in such a manner as to be engaged with each other according to the third embodiment not covered by the present invention;
FIG. 30 is an exemplary screen view illustrating a region where a surface of the temporary crown and a surface of the antagonist tooth are brought into contact with each other according to the third embodiment not covered by the present invention;
FIG. 31 is an exemplary screen view illustrating a candidate occlusal adjustment polygon group according to the third embodiment not covered by the present invention;
FIG. 32 is an exemplary screen view illustrating a composite vector according to the third embodiment not covered by the present invention;
FIG. 33 is an exemplary screen view illustrating the optimal occlusal adjustment region according to the third embodiment not covered by the present invention;
FIG. 34 is an exemplary screen view illustrating a plurality of polygons selected in a manner that corresponds to a region where the temporary crown and the antagonist tooth are engaged with each other and the candidate occlusal adjustment polygon group according to the third embodiment not covered by the present invention; and
FIG. 35 is an exemplary screen view illustrating the optimal occlusal adjustment region made up of a combination of a plurality of polygons according to the third embodiment not covered by the present invention.

### Detailed Description of Exemplary Embodiments

A method of forming, by utilizing a computer, an implant shape for minimizing stress to be applied to an implant according to an aspect of the present invention includes: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle.

A method of forming, by utilizing a computer, an implant shape for minimizing stress to be applied to an implant according to another aspect of the present invention includes: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle, wherein in a case where the value of the single vector derived in the vector derivation step S8 is such that the angle R with respect to the axis of the virtual implant 100 falls outside a range from 0 degrees to 10 degrees, the occlusal adjustment point 201b of the second temporary crown 200b is redetermined on the basis of an input from a user, and the steps S6 and S7 are reiterated.

A method of forming, by utilizing a computer, an implant shape for minimizing stress to be applied to an implant according to still another aspect of the present invention includes: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle, wherein a crown shape of the first temporary crown 200a in the first temporary crown arrangement step S4 is determined using positional information of a missing tooth, and the second temporary crown 200b in the second temporary crown substitution step S5 is provided in such a manner that a size of the second temporary crown 200b is adjusted considering a relationship between each of the adjacent teeth 12 and 13 positioned to the left and right, respectively, of the missing tooth and the antagonist tooth 21 engaged with the missing tooth.

A method of forming, by utilizing a computer, an implant shape for minimizing stress to be applied to an implant according to still another aspect of the present invention includes: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle, wherein a crown shape of the first temporary crown 200a in the first temporary crown arrangement step S4 is determined using positional information of a missing tooth, the second temporary crown 200b in the second temporary crown substitution step S5 is provided in such a manner that a size of the second temporary crown 200b is adjusted considering a relationship between each of the adjacent teeth 12 and 13 positioned to the left and right, respectively, of the missing tooth and the antagonist tooth 21 engaged with the missing tooth, and wherein a crown shape of the first temporary crown 200a in the first temporary crown arrangement step S4 is determined using positional information of a missing tooth, and the second temporary crown 200b in the second temporary crown substitution step S5 is provided in such a manner that a size of the second temporary crown 200b is adjusted considering a relationship between each of the adjacent teeth 12 and 13 positioned to the left and right, respectively, of the missing tooth and the antagonist tooth 21 engaged with the missing tooth.

A method of forming, by utilizing a computer, an implant shape for minimizing stress to be applied to an implant according to still another aspect of the present invention includes: a tooth scan model generation step S1 of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data; a scan file alignment step S2 of aligning a three-dimensional model 10 for the treatment-target region and a three-dimensional model 20 for the teeth arrangement facing the treatment-target region in such a manner as to be engaged with each other, the model 10 and the model 20 being generated in the tooth scan model generation step S1; a virtual implant arrangement step S3 of positioning a virtual implant 100 in a missing-tooth portion 11 of the three-dimensional model 10 for the treatment-target region, the model 10 being generated in the tooth scan model generation step S1; a first temporary crown arrangement step S4 of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100; a second temporary crown substitution step S5 of substituting a second temporary crown 200b for the first temporary crown 200a arranged in the first temporary crown arrangement step S4 on the basis of a relationship between each of the adjacent teeth 12 and 13 adjacent to the missing-tooth portion 11 and an antagonist tooth 21 corresponding to the missing-tooth portion 11; an occlusal adjustment point extraction step S6 of extracting an occlusal adjustment point 201b at which the second temporary crown 200b is engaged with a missing-tooth-engaged antagonist tooth 21, after the second temporary crown substitution step S5; a second temporary crown occlusal adjustment region derivation step S7 of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6; a vector derivation step S8 of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other; and a crown shape determination step S9 of determining a shape of the second temporary crown 200b in a case where a value of the single vector derived in the vector derivation step S8 is such that an angle R with respect to an axis of the virtual implant 100 is equal to or smaller than a predetermined angle, wherein three to eight occlusal adjustment points 201b in the occlusal adjustment point extraction step S6 are extracted.

According to an embodiment not covered by the present invention, a method of recommending an optimal occlusal adjustment region for an implant by utilizing a computer includes: a step of generating a three dimensional model for each of a treatment-target region including a missing-tooth portion and an antagonist tooth region facing the treatment-target region from scan data generated by scanning a patient's oral cavity using an intraoral scanner; a step of arranging a virtual implant abutment in a missing-tooth portion of the three-dimensional model for the treatment-target region; a step of selecting any one tooth model from among a plurally of tooth models as prestored three-dimensional models and setting the selected one tooth model to be a temporary crown; a step of adjusting a size of the temporary crown according to shapes of adjacent teeth adjacent to the missing-tooth portion in the three-dimensional model for the treatment-target region and to a shape of an antagonist tooth corresponding to the missing-tooth portion in the three-dimensional model for the antagonist tooth region and arranging the temporary crown on the top of the virtual implant abutment; a step of generating an optimal occlusal adjustment region where a direction of a composite vector based on orthogonal vectors applied from the antagonist tooth to the temporary crown is toward a lower cross-sectional region of the virtual implant abutment, from the three-dimensional model for the antagonist tooth on the basis of at least two regions where a surface of the temporary crown and a surface of the antagonist tooth are brought into contact with each other, in a case where the temporary crown arranged on the top of the virtual implant abutment and the antagonist tooth are aligned in such a manner as to be engaged with each other; a step of generating an optimal occlusal adjustment region image for displaying the optimal occlusal adjustment region on the three-dimensional model for the antagonist tooth and an antagonist tooth region image for displaying the three-dimensional model for the antagonist tooth region; and a step of displaying the optimal occlusal adjustment region image on the antagonist tooth region image in an overlapping manner.

In the method, in the step of generating a three-dimensional mode, the three-dimensional model made up of a combination of a plurality of polygons for the treatment-target region and a combination of a plurality of polygons for the antagonist tooth region. In the method, in the step of generating the optimal occlusal adjustment region, in a case where the temporary crown and the antagonist tooth are aligned in such a manner as to be engaged with each other, a plurality of polygons where the direction of the composite vector based on the orthogonal vectors orthogonal to respective surfaces of a preset number or greater number of a plurality of polygons adjacent to a plurality of polygons belonging to the three-dimensional model for the antagonist tooth corresponding to at least two regions where the surface of the temporary crown and the surface of the antagonist tooth are brought into contact with each other is toward the lower cross-sectional region of the virtual implant abutment are selected, and thus the optimal occlusal adjustment region is generated.

The step of generating an optimal occlusal adjustment region includes: a step of selecting a plurality of polygons belonging to the three dimensional model for the antagonist tooth corresponding to at least two regions where the surface of the temporary crown and the surface of the antagonist tooth are brought into contact with each other in the case where the temporary crown and the antagonist tooth are aligned in such a manner as to be engaged with each other; a step of selecting a preset number or greater number of a plurality of polygons adjacent to the plurality of polygons selected and setting a candidate occlusal adjustment polygon group on a per-region basis; a step of selecting at least one polygon on a per-candidate occlusal adjustment polygon group basis in such a manner that the direction of the composite vector based on the orthogonal vectors orthogonal to respective surfaces of a plurality of polygons belonging to each of the different candidate occlusal adjustment polygon groups is toward a lower cross-sectional region of the virtual implant abutment; and a step of generating an optimal occlusal adjustment region made up of a combination of polygons selected on a per-candidate occlusal adjustment polygon group basis.

In the step of selecting any one tooth model from among a plurally of tooth models as prestored three-dimensional models and setting the selected one tooth model to be a temporary crown, any one tooth model corresponding to a position of the missing-tooth portion is selected from among the plurality of tooth models, as the prestored three-dimensional models, according to the position of the missing-tooth portion, and the selected one tooth model is set to be a temporary crown.

According to another embodiment not covered by the present invention, an apparatus for recommending an optimal occlusal adjustment region for an implant by utilizing a computer includes: a scan data input unit into which scan data generated by scanning a patient's oral cavity using an intraoral scanner is input from the outside; a three-dimensional model generation unit generating a three-dimensional model for each of a treatment-target region including a mission-tooth portion and an antagonist tooth region facing the treatment-target region from the scan data; a first arrangement unit arranging a virtual implant abutment in the missing-tooth portion of the three-dimensional model for the treatment-target model; a temporary crown setting unit selecting any one tooth model from among a plurality of tooth models as prestored three-dimensional models and setting the selected one tooth model as a temporary crown; a second arrangement unit adjusting a size of the temporary crown according to shapes of adjacent teeth adjacent to the missing-tooth portion in the three-dimensional model for the treatment-target region and to a shape of an antagonist tooth corresponding to the missing-tooth portion in the three-dimensional model for the antagonist tooth region and arranging the temporary crown on the top of the virtual implant abutment; an optimal occlusal adjustment region generation unit generating an optimal occlusal adjustment region where a direction of a composite vector based on orthogonal vectors applied from the antagonist tooth to the temporary crown is toward a lower cross-sectional region of the virtual implant abutment, from the three-dimensional model for the antagonist tooth on the basis of at least two regions where a surface of the temporary crown and a surface of the antagonist tooth are brought into contact with each other, in a case where the temporary crown and the antagonist tooth are aligned in such a manner as to be engaged with each other on the top of the virtual implant abutment; an image generation unit generating an optimal occlusal adjustment region image for displaying the optimal occlusal adjustment region on the three-dimensional model for the antagonist tooth and an antagonist tooth region image for displaying the three-dimensional model for the antagonist tooth region; and a display unit displaying the optimal occlusal adjustment region image on the antagonist tooth region image in an overlapping manner.

The three-dimensional model generation unit generates the three-dimensional model made up of a combination of a plurality of polygons for the treatment-target region and a combination of a plurality of polygons for the antagonist tooth region. In a case where the temporary crown and the antagonist tooth are aligned in such a manner as to be engaged with each other, the optimal occlusal adjustment region generation unit selects a plurality of polygons where the direction of the composite vector, based on the orthogonal vectors orthogonal to respective surfaces of a preset number or greater number of a plurality of polygons adjacent to a plurality of polygons belonging to the three-dimensional model for the antagonist tooth corresponding to at least two regions where the surface of the temporary crown and the surface of the antagonist tooth are brought into contact with each other, is toward the lower cross-sectional region of the virtual implant abutment and thus generates the optimal occlusal adjustment region.

In the case where the temporary crown and the antagonist tooth are aligned in such a manner as to be engaged with each other, the optimal occlusal adjustment region generation unit selects a plurality of polygons belonging to the three dimensional model for the antagonist tooth corresponding to at least two regions where the surface of the temporary crown and the surface of the antagonist tooth are brought into contact with each other, selects a preset number or greater number of a plurality of polygons adjacent to the plurality of polygons selected, sets a candidate occlusal adjustment polygon group on a per-region basis, selects at least one polygon on a per-candidate occlusal adjustment polygon group basis in such a manner that a direction of a composite vector based on orthogonal vectors orthogonal to respective surfaces of a plurality of polygons belonging to each of the different candidate occlusal adjustment polygon groups is toward a lower cross-sectional region of the virtual implant abutment, and generates an optimal occlusal adjustment region made up of a combination of polygons selected on a per-candidate occlusal adjustment polygon group basis.

The temporary crown setting unit selects any one tooth model corresponding to a position of the missing-tooth portion from among a plurality of tooth models, as prestored three-dimensional models, according to the position of the missing-tooth portion, and sets the selected one tooth model to be a temporary crown.

Advantages and features of the present invention, and methods of achieving the advantages and the features will be apparent from the accompanying drawings and from embodiments that will be described in detail below. However, the present invention is not limited to the embodiments that will be disclosed below and can be practiced in various different forms. The embodiments are only provided to make a complete disclosure of the present invention and to provide full notice of the scope of the present invention to a person of ordinary skill in the art to which the present invention pertains. The scope of the present invention is defined by the appended claims. Throughout the specification, the terms used for describing embodiments do not impose any limitation on the present invention. Unless specified otherwise through the present specification, a singular noun or a singular noun phrase may have a plural meaning.

The term prosthesis, used to describe the present invention in detail, means an artificial replacement for one teeth or two or more teeth or for tooth-related tissue. In addition, types of prostheses include an inlay, an onlay, a crown, a laminate, a bridge, a coping, an implant, a denture, a surgical guide, and the like.

In addition, as an example, in a case where the prosthesis is an implant, the prosthesis may be defined as including at least one of an implant fixture inserted into the alveolar bone, an implant abutment connected to the implant fixture, and an implant crown covering the top of the implant abutment and forming an exterior upper portion of an artificial tooth.

Data may be transmitted and received through wireless/wired communication among apparatuses that are used while performing a method of forming, by utilizing a computer, an implant shape for minimizing stress to be applied to an implant according to an embodiment of the present invention. For example, wireless communication standards, such as wireless LAN (WLAN), Wi-Fi, Wibro, Wimax, High Speed Downlink Packet Access (HSDPA), may be used. However, the apparatuses are not limited to these wireless communication standards and may use wired communication standards, such as Universal Serial Bus, Ethernet, xDSL (ADSL or VDSL), Hybrid Fiber Coaxial Cable (HFC), Fiber to the Curb (FTTC), and Fiber to the Home (FTTH), depending on how a system is realized. In addition, short-distance communication standards, such as Bluetooth, Radio Frequency Identification (RFID), Infrared Data Association (IrDA), Ultra-Wideband (UWB), ZigBee, and Near Field Communication (NFC), may be used.

First, according to the present invention, an intraoral scanner generating scan data scans a patient's oral cavity and generates scan data. For example, the intraoral scanner scans a patient's treatment-target region and generates the scan data. The treatment-target region may be a region on which surgery for prosthesis is performed, among regions of the patient's oral cavity and may be a region including the region on which the surgery for prosthesis is performed and may be a region where adjacent teeth adjacent to the region on which the surgery for prosthesis is performed are positioned.

The present invention relates to a method of forming, by utilizing a computer, an implant shape for minimizing stress to be applied to an implant and, more particularly, to a method of forming an implant shape for minimizing stress to be applied to an implant by changing a shape of an implant crown in such a manner that an angle between a single vector, in which forces applied by an antagonist tooth to a contact surface of an implant are equivalent to each other and in which moments applied by the antagonist tooth thereto are equivalent to each other, and an implant approaches 0.

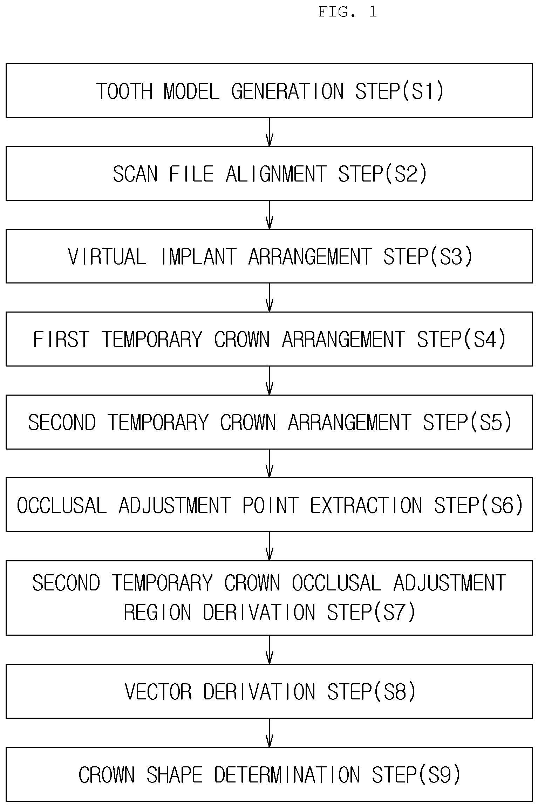

FIG. 1 is a diagram illustrating the order of steps of a method of forming, by utilizing a computer, an implant shape for minimizing stress to be applied to an implant according to the present invention. The method according to the present invention is configured to include a tooth scan model generation step S1, a scan file alignment step S2, a virtual implant arrangement step S3, a first temporary crown arrangement step S4, a second temporary crown substitution step S5, an occlusal adjustment point extraction step S6, a second temporary crown occlusal adjustment region derivation step S7, a vector derivation step S8, and a crown shape determination step S9.

Each of the steps is described in detail as follows.

### S1) Tooth Scan Model Generation Step

The tooth scan model generation step S1 is a step of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data.

In the tooth scan model generation step S1, a tooth in the treatment-target region and the teeth arrangement facing the treatment-target region is scanned, and the three-dimensional model for each of the treatment-target region and the teeth arrangement is generated.

In the tooth scan model generation step S1, the scan data generated by scanning a patent's oral cavity using the intraoral scanner is received, and the three-dimensional model for the treatment-target region is generated on the basis of the received scan data. In addition, in the tooth scan model generation step S1, basic information (a name, a visiting date, a medical record, and the like) of the patient may be received.

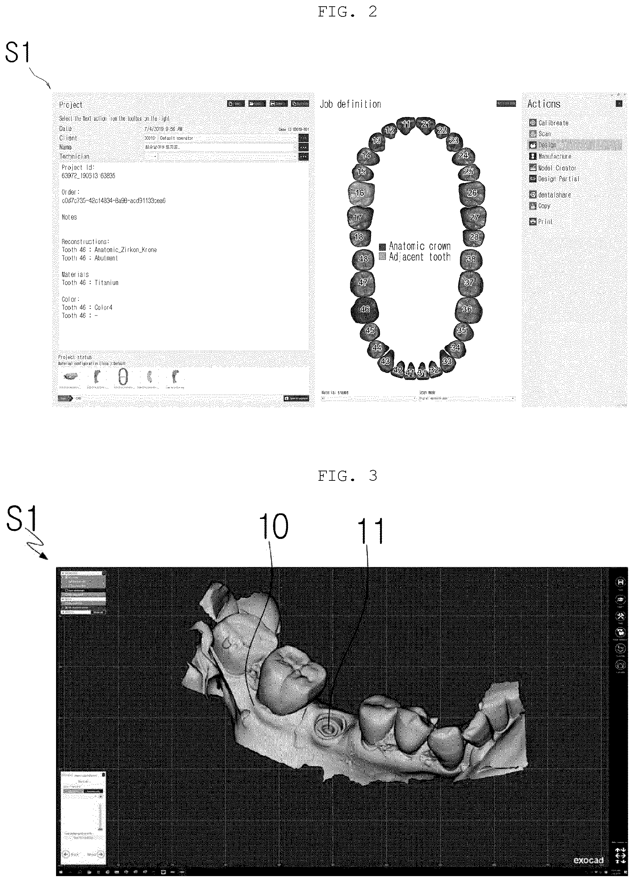

FIGS. 2 to 4 are screen views each illustrating the tooth scan model generation step S1. FIG. 2 illustrates an exemplary screen from which the basic information of the patient is input. FIG. 3 illustrates an exemplary screen on which a three-dimensional model 10 generated for the treatment-target region on the basis of the scan data is displayed. The three-dimensional model 10 includes a missing-tooth portion 11.

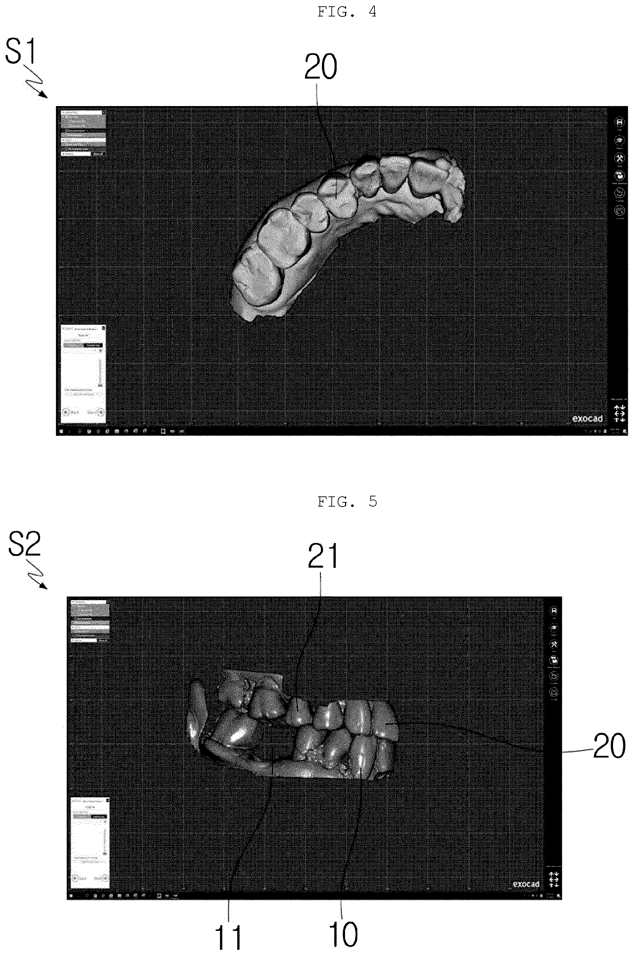

FIG. 4 illustrates a state where a 3D scan file of the teeth arrangement facing the treatment-target region is input. That is, FIG. 4 is an exemplary screen on which a three-dimensional model 20 for the teeth arrangement facing the treatment-target region is displayed. The three-dimensional model 20 includes a missing-tooth-engaged antagonist tooth 21 positioned in such a manner as to be engaged with the missing-tooth portion 11.

FIG. 2 illustrates that the basic information of the patient is input and that information No. 46 on a missing tooth and information No. 16 on a tooth coming into contact with the missing tooth are marked in different colors.

### S2) Scan File Alignment Step

The scan file alignment step S2 is a step of aligning the three-dimensional model 10 for the treatment-target region and the three-dimensional model 20 for the teeth arrangement facing the treatment-target region in such a manner as to be engaged with each other, the model 10 and the model 20 being generated in the tooth scan model generation step S1.

FIG. 5 is an exemplary screen illustrating that in the scan file alignment step S2, the treatment-target region and the teeth arrangement facing the treatment-target region are aligned in such a manner as to be engaged with each other using maxillary and mandibular occlusal adjustment information. In FIG. 5, the treatment-target region is positioned in the mandibula, and the teeth arrangement facing the treatment-target region is positioned in the maxilla.

### S3) Virtual Implant Arrangement Step

The virtual implant arrangement step S3 is a step of positioning a virtual implant 100 in the missing-tooth portion 11 of the generated three-dimensional model 10 for the treatment-target region.

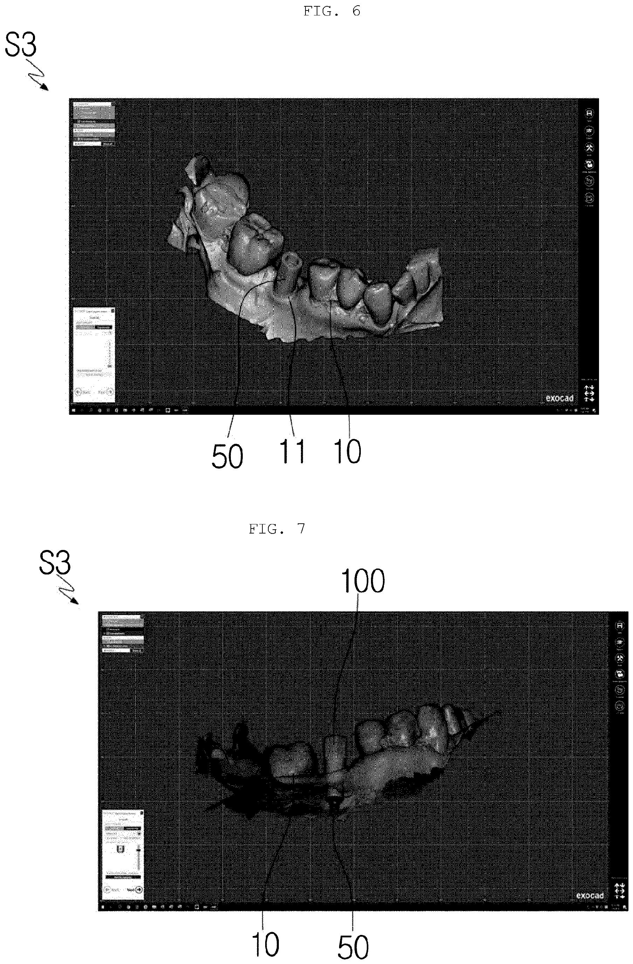

The virtual implant arrangement step S3 is described in detail below with reference to FIGS. 6 to 8.

With reference to FIG. 6, a scan file of a tooth to which an implant scan body is fastened is aligned for information on a position at which a virtual implant is to be arranged.

With reference to FIG. 7, positional information of an upper connection portion of an implant inside an actual bone may be known by superimposing the virtual implant 100 on an implant scan body 50.

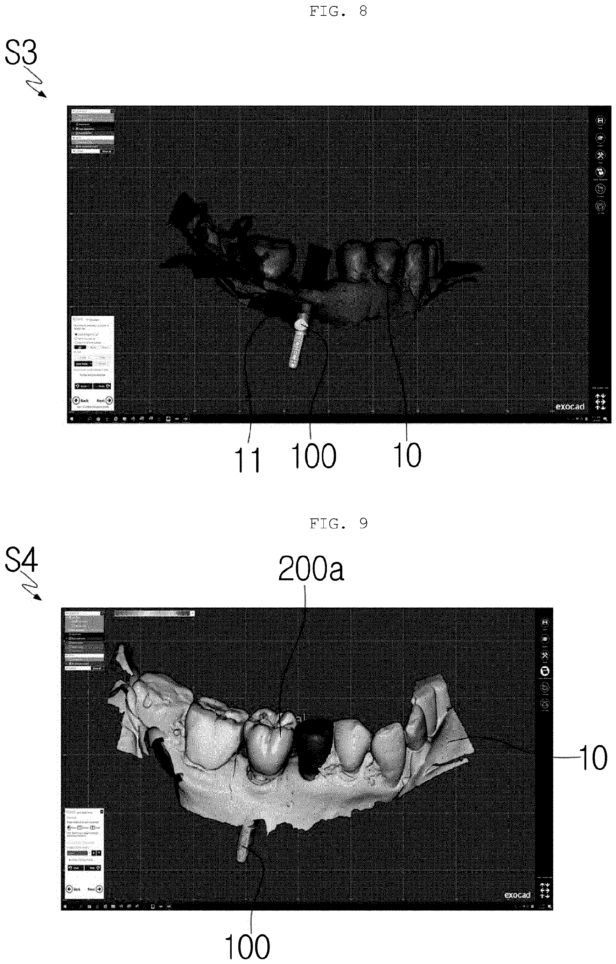

FIG. 8 illustrates that the virtual implant 100 is positioned in the missing-tooth portion 11 on the basis of the positional information of the upper connection portion of the actual implant that is known in FIG. 7.

### S4) First Temporary Crown Arrangement Step

The first temporary crown arrangement step S4 is a step of automatically arranging an arbitrarily selected first temporary crown 200a on the virtual implant 100 arranged in the virtual implant arrangement step S3 and connecting the arbitrarily selected first temporary crown 200a to a connection portion 110 of the virtual implant 100.

The first temporary crown 200a arranged in the first temporary crown arrangement step S4 has one of crown shapes stored in a tooth library. The tooth library serves to store shapes of teeth categorized according to tooth positions. The shapes of teeth on a per-tooth position basis that are usually used for dental treatment are stored.

In addition, the tooth library may include a plurality of tooth models having different occlusal adjustment surface information. The tooth models may be categorized according to user age groups, tooth shapes, tooth sizes, tooth worn-portions, worn-tooth patterns, worn-tooth areas, and the like.

The occlusal adjustment surface information here may include information on the extent to which a tooth is deformed. The information on the extent includes information on a wearing position and a wearing pattern that occur around an occlusal adjustment point of a tooth due to the use of the tooth and the like, or information on tooth shapes, tooth sizes and the like. Examples of the tooth deformation may include a tooth that has a tooth occlusal adjustment surface which is almost not worn when compared with a standard tooth, a tooth that has a tooth occlusal adjustment surface which is worn in a rectangle-shaped or small ellipse-shaped wear pattern that occurs around a tooth occlusal adjustment point, a tooth that has a tooth occlusal adjustment surface which is worn in an isosceles triangle-shaped or wide ellipse-shaped wear pattern that occurs around the tooth occlusal adjustment point, and the like. The rectangle-shaped, isosceles triangle-shaped, and ellipse-shaped wear patterns are only exemplary. The tooth deformation recognizable by a program according to the present invention and the tooth deformation of the tooth model stored in the tooth library are not limited to these patterns.

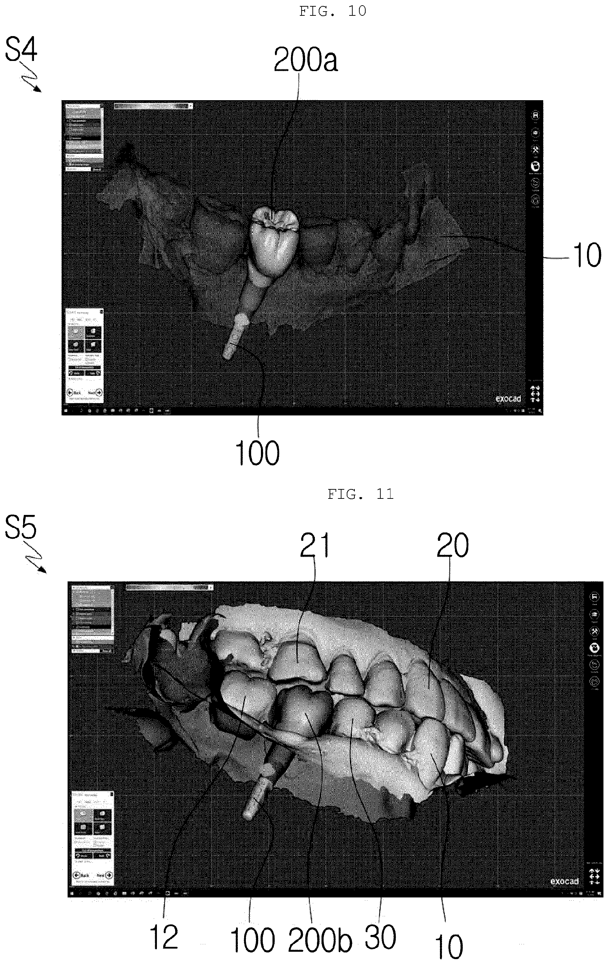

FIGS. 9 and 10 are exemplary screen views each illustrating a state where the first temporary crown 200a is arranged on the virtual implant 100.

### S5) Second Temporary Crown Substitution Step

The second temporary crown substitution step S5 is a step of substituting a second temporary crown 200b for the first temporary crown 200a arranged in the first temporary crown arrangement step S4 on the basis of a relationship between each of the adjacent 12 and 13 adjacent to the missing-tooth portion 11 and an antagonist tooth 21 corresponding to the missing-tooth portion 11.

The adjacent teeth 12 and 13 mean teeth positioned adjacent to the opposite sides, respectively, of the tooth-missing portion, and the antagonist tooth 21 means a tooth engaged with the tooth-missing portion.

FIG. 11 is a screen view illustrating the second temporary crown substitution step S5. When a crown shape of the first temporary crown 200a is determined using only positional information of a missing tooth, the second temporary crown 200b is provided in such a manner that a size of the second temporary crown 200b is adjusted considering not only positional information of the missing tooth, but also a relationship between each of the adjacent teeth 12 and 13 positioned to the left and right, respectively, of the missing tooth and the antagonist tooth 21 engaged with the missing tooth.

That is, when a width in the leftward-rightward direction of the first temporary crown 200a arranged at a missing-tooth position is greater than a with between the first adjacent teeth 12 and the second adjacent teeth 13, the width in the leftward-rightward direction may be decreased considering respective positions of the first and second adjacent teeth 12 and 13. When the first temporary crown 200a is engaged with the missing-tooth-engaged antagonist tooth 21, if a height of the first temporary crown 200a is so low that the first temporary crown 200a cannot be engaged with the missing-tooth-engaged antagonist tooth 21, a height of the first temporary crown 200a is adjusted, and thus a shape of the second temporary crown 200b is provided.

### S6) Occlusal Adjustment Point Extraction Step

The occlusal adjustment point extraction step S6 is a step of extracting an occlusal adjustment point 201b at which the second temporary crown 200b is engaged with a missing-tooth-engaged antagonist tooth 21, after the second temporary crown substitution step S5.

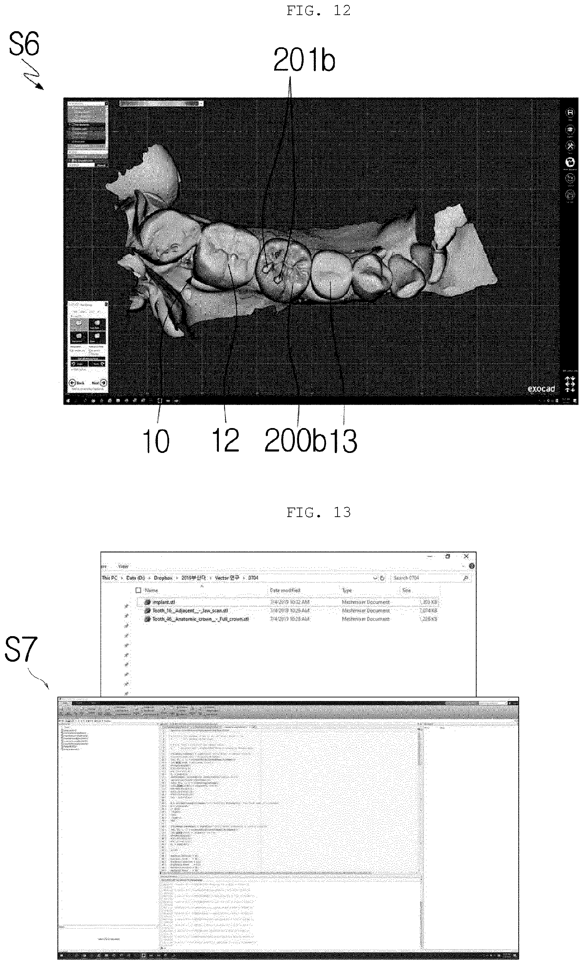

FIG. 12 is an exemplary screen view illustrating the occlusal adjustment point extraction step S6. From FIG. 12, it can be seen that three occlusal adjustment points 201b marked in a blue color are extracted. It is desirable that three to eight occlusal adjustment points may be extracted.

### S7) Second Temporary Crown Occlusal Adjustment Region Derivation Step

The second temporary crown occlusal adjustment region derivation step S7 is a step of deriving an occlusal adjustment region 202b of the second temporary crown 200b that is a surface remaining after subtracting a volume of the antagonist tooth 21 from a volume of the second temporary crown 200b, in a state where the three-dimensional model 10 for the treatment-target region including the second temporary crown 200b and the three-dimensional model 20 for the teeth arrangement facing the treatment-target region including the antagonist tooth 21 are engaged with each other, after the occlusal adjustment point extraction step S6.

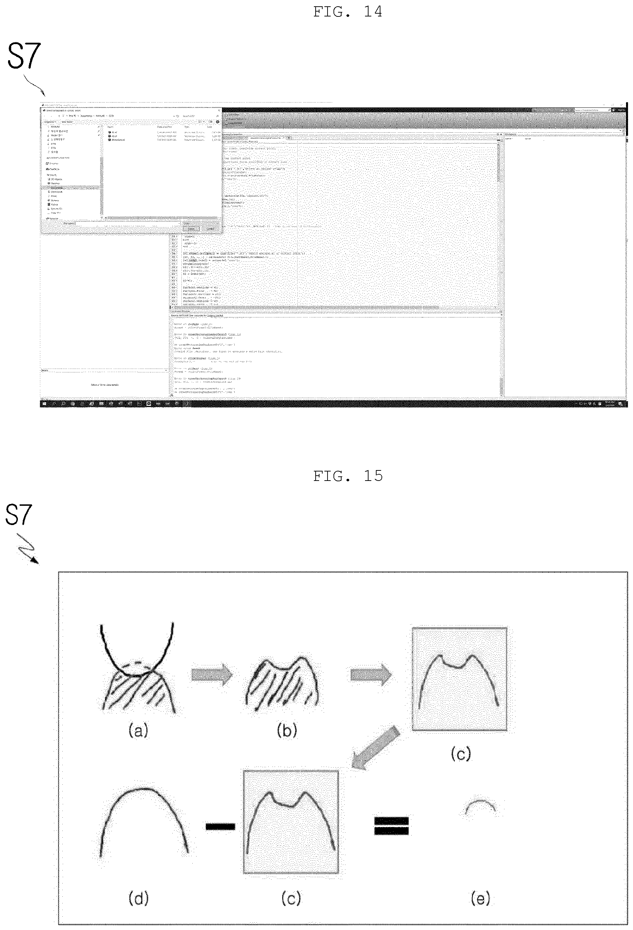

FIGS. 13 and 14 are exemplary screen views each illustrating that a vector-value analysis code is executed in order to analyze a vector value in a subsequent step.

FIG. 15 is a screen view illustrating the second temporary crown occlusal adjustment region derivation step S7. When respective overlapping portions of the second temporary crown 200b and the antagonist tooth 21 are removed as illustrated in FIG. 15(a), the resulting shape of the second temporary crown 200b is as illustrated in FIG. 15(b). FIG. 15(c) illustrates only a surface of the second temporary crown 200b whose portion overlapping the portion of the antagonist tooth 21 is removed. FIG. 15(d) illustrates only a surface of the second temporary crown 200b. When respective overlapping portions of FIG. 15(d) and FIG. 15(c) illustrating the second temporary crown 200b whose portion overlapping the portion of the antagonist tooth 21 is removed are removed, the resulting surface of the second temporary crown 200b on which the occlusal adjustment points are positioned is obtained as illustrated in FIG. 15(e).

In the occlusal adjustment point extraction step S6, only respective occlusally adjusted portions of the second temporary crown 200b and the antagonist tooth 21 are simply displayed. However, in the second temporary crown occlusal adjustment region derivation step S7, a volume of a portion of the second temporary crown 200b that is removed to be adjusted occlusally with respect to the antagonist tooth 21 may be obtained.

### S8) Vector Derivation Step

The vector derivation step S8 is a step of deriving a single vector in which external force vectors applied by the antagonist tooth 21 to all local portions in the occlusal adjustment region 202b of the second temporary crown 200b derived in the second temporary crown occlusal adjustment region derivation step S7 are equivalent to each other.

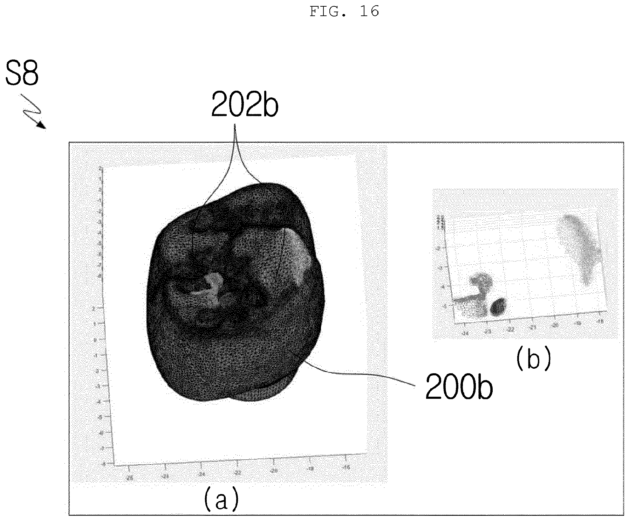

FIG. 16 is a screen view illustrating the occlusal adjustment region 202b of the second temporary crown 200b derived in the second temporary crown occlusal adjustment region derivation step S7.

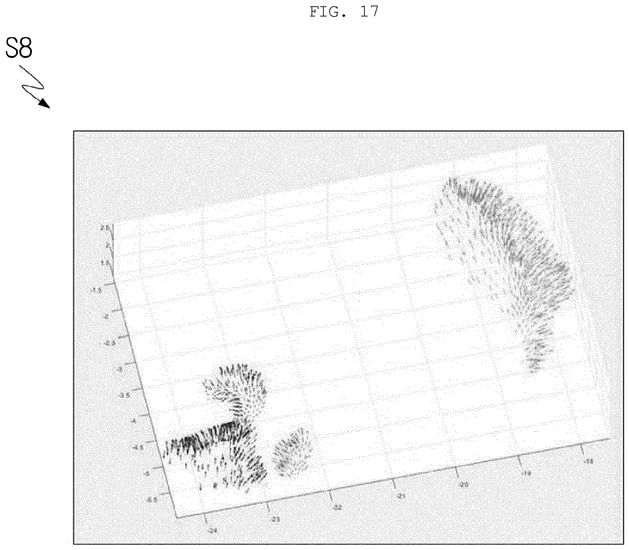

FIG. 17 is a screen view illustrating a state where forces applied from points on the occlusal adjustment region 202b illustrated in FIG. 16(a) to the antagonist tooth 21 are expressed on a per-vector basis.

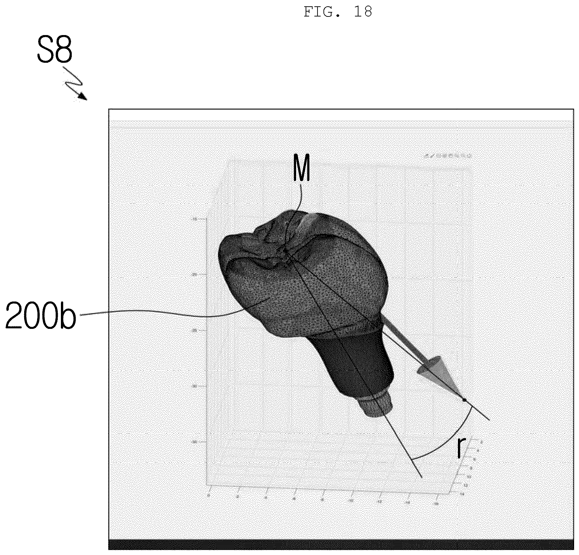

In FIG. 18, an expression as a single vector is written in such a manner that forces applied by the antagonist tooth 21 to the occlusal adjustment region 202b of the second temporary crown 200b are equivalent to each other and that rotational moments applied by the antagonist tooth 21 thereto are equivalent to each other. In FIG. 18, a position of an application point M and a magnitude of a force are expressed. When an angle R between an implant structure and a single vector is close to 0, the implant structure is not damaged. The greater the angle R, the more the implant structure is damaged by the rotational moment.

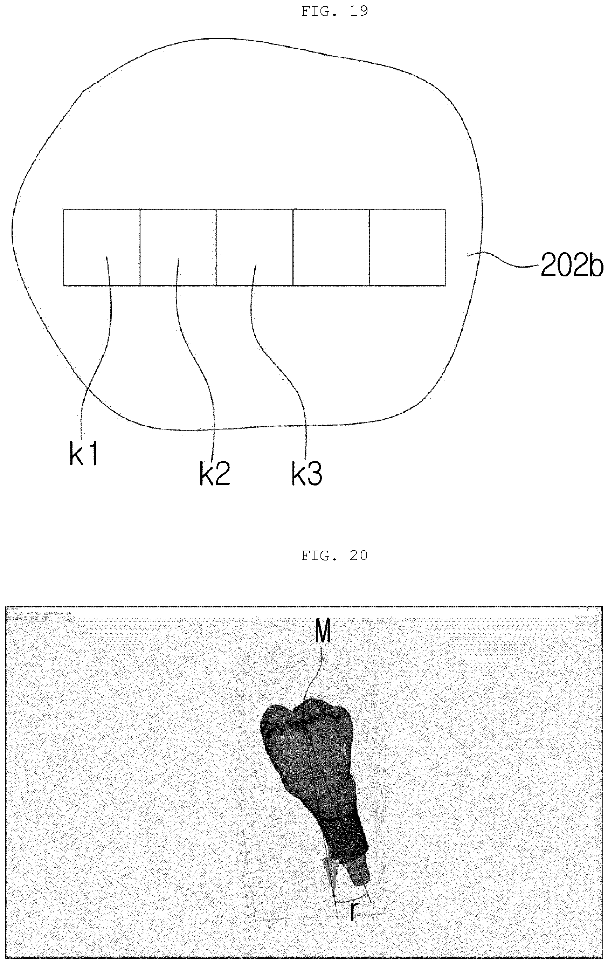

In addition, with reference to FIG. 19, one occlusal adjustment region 202b are cut into elements, and thus an area of the occlusal adjustment region 202b is divided into sub-areas K1, K2, K3, and so forth. Then, on the assumption that the same vector is applied to each of the sub-areas, an external force vector of the occlusal adjustment region 202b may be computed.

When a value of the angle R does not fall within a range of 0 degrees to 10 degrees in FIG. 18, the occlusal adjustment point 201b of the second temporary crown 200b may be reselected, and Steps S6 to S8 may be reiterated. The value of the angle R may approach 0 as in FIG. 20 while Steps S6 to S8 are reiterated.

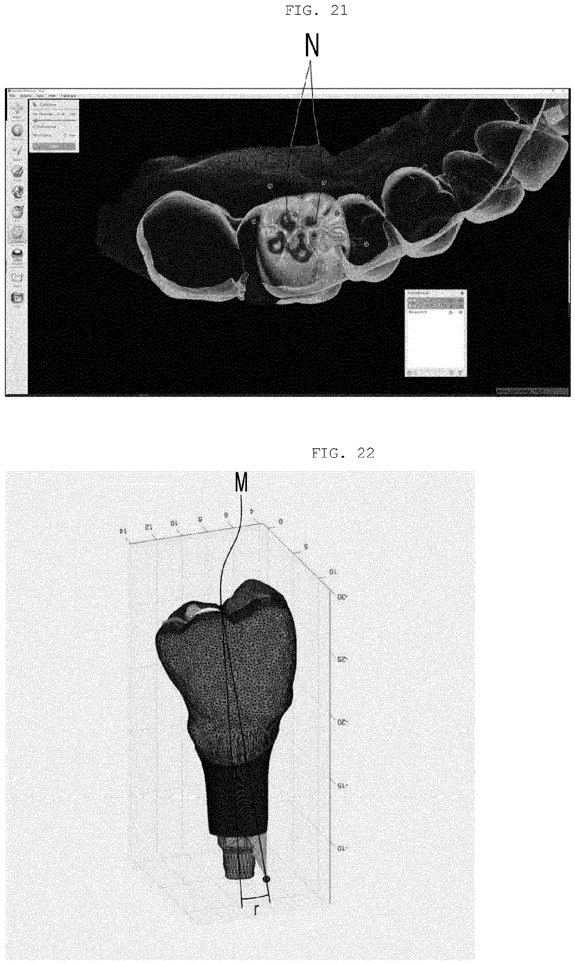

FIG. 21 illustrates a state where in a case where the value of the angle R in FIG. 18 falls outside the range of 0 degrees to 10 degrees, the occlusal adjustment point 201b of the second temporary crown 200b is redetermined (refer to occlusal adjustment points indicated by the letter N in FIG. 21).

At this point, a method of redetermining the occlusal adjustment point 201b is performed as follows. The occlusal adjustment point is determined by changing the shape of the second temporary crown 200b on the basis of the value of the angle R in FIG. 18. Alternatively, the occlusal adjustment point may be redetermined on the basis of an input from the user or according to a program executed by a processor.

FIG. 22 illustrates a state where the value of the angle R is recomputed by reiterating Steps S7 and S8 to redetermine the occlusal adjustment point. From FIG. 22, it can be seen that the value of the angle R is closer to 0 than in FIG. 18.

### S9) Crown Shape Determination Step

The crown shape determination step S9 is a step of determining a shape of the second temporary crown 200b in a case where a value of the single vector derived in the vector derivation step S8 is such that an angle R with respect to an axis of the virtual implant 100 is equal to or smaller than a predetermined angle.

An apparatus for and a method of recommending an optimal occlusal adjustment region for an implant according to second and third embodiments not covered by the present invention will be described below with reference to the drawings.

The optimal occlusal adjustment region for an implant is generated, and the generated optimal occlusal adjustment region is displayed on the antagonist tooth. Accordingly, without separate analysis of stress, the direction of stress applied to the implant is toward the center of respective surfaces of a fixture and an abutment that are combined with each other. Thus, there is provided an advantage in that a crown can be designed in such a manner as to minimize the stress to be applied to the implant.

The implant is configured to include a fixture inserted into the alveolar bone, an abutment connected to an upper portion of the fixture, and a crown connected to an upper portion of the abutment and forming an exterior upper portion of an artificial tooth.

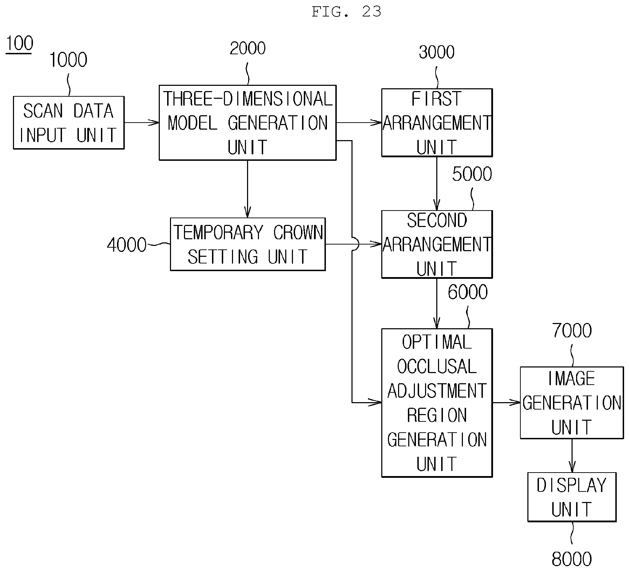

FIG. 23 is a block diagram illustrating the apparatus for recommending an optimal occlusal adjustment region for an implant by utilizing a computer according to the second embodiment not covered by the present invention.

With reference to FIG. 23, an apparatus 1 for recommending an optimal occlusal adjustment region for an implant according to the second embodiment not covered by the present invention may include a scan data input unit 1000, a three-dimensional model generation unit 2000, a first arrangement unit 3000, a temporary crown setting unit 4000, a second arrangement unit 5000, an optimal occlusal adjustment region generation unit 6000, an image generation unit 7000, and a display unit 8000.

Scan data generated by scanning a patient's oral cavity using an intraoral scanner is input from the outside into the scan data input unit 1000.

The scan data generated by scanning the patient's oral cavity in which a scan body is fastened to the tooth-missing portion are input from the outside into the scan data input unit 1000.

The scan body here serves to specify a position of an implant fixture implanted in the tooth-missing portion. The scan body means a structure that is combined with the top of the implant fixture implanted in the tooth-missing portion and protrudes outward from the gum.

The three-dimensional model generation unit 2000 may generate a three-dimensional model for each of a treatment-target region including a scan body and an antagonist tooth region facing the treatment-target region from the scan data that are input into the scan data input unit 1000.

The three-dimensional model generation unit 2000 may generate from the scan data the three-dimensional model made up of a combination of a plurality of polygons for the treatment-target region and a combination of a plurality of polygons for the antagonist tooth region.

The first arrangement unit 3000 may arrange a virtual implant abutment in the missing-tooth portion of the three-dimensional model for the treatment-target region on the basis of a position of the scan body.

The first arrangement unit 3000 may arrange a preset virtual implant fixture according to the position of the scan body and may arrange the virtual implant abutment on the top of the arranged virtual implant fixture in such a manner that a lower end of the virtual implant abutment is engaged with an upper end of the virtual implant fixture.

The temporary crown setting unit 4000 may select any one tooth model from among a plurality of tooth models as prestored three-dimensional models and may set the selected one tooth model to be a temporary crown.

The temporary crown setting unit 4000 may select any one tooth model corresponding to a position of the tooth-missing portion from among the plurality of tooth models as prestored three-dimensional models and may set the selected one tooth model to be a temporary crown.

The second arrangement unit 5000 may adjusting a size of the temporary crown according to shapes of adjacent teeth adjacent to the missing-tooth portion in the three-dimensional model for the treatment-target region and to a shape of an antagonist tooth corresponding to the missing-tooth portion in the three-dimensional model for the antagonist tooth region and may arrange the temporary crown on the top of the virtual implant abutment.

At this point, the second arrangement unit 5000 may compute a width between the temporary crown and a tooth adjacent to the crown tooth on the basis of the selected tooth model, may adjust the size of the temporary crown in a manner that increases or decreases the size of the temporary crown within a range where the temporary crown does not interfere with the adjacent tooth, and may arrange the temporary crown on the top of the virtual implant abutment.

Alternatively, the second arrangement unit 5000 may compute a width between the temporary crown and the tooth adjacent to the crown tooth on the basis of the selected tooth model, may set the size of the temporary crown in such a manner that the temporary crown has a maximum size within a range where the temporary crown does not interfere with the adjacent tooth, and may arrange the temporary crown on the top of the virtual implant abutment.

In a case where the temporary crown and the antagonist tooth are aligned on the top of the virtual implant abutment in such a manner as to be engaged with each other, the optimal occlusal adjustment region generation unit 6000 may select a combination of polygons where a direction of a composite vector based on orthogonal vectors applied from the antagonist tooth to the temporary crown is toward a lower cross-sectional region of the virtual implant abutment, from the three-dimensional model for the antagonist tooth on the basis of at least two regions where a surface of the temporary crown and a surface of the antagonist tooth are brought into contact with each other, and thus may generate an optimal occlusal adjustment region.

In the case where the temporary crown and the antagonist tooth are aligned in such a manner as to be engaged with each other, the optimal occlusal adjustment region generation unit 6000 may select a combination of polygons where the direction of the composite vector, based on the orthogonal vectors orthogonal to respective surfaces of a preset number or greater number of a plurality of polygons adjacent to a plurality of polygons belonging to the three-dimensional model for the antagonist tooth corresponding to at least two regions where the surface of the temporary crown and the surface of the antagonist tooth are brought into contact with each other, is toward the lower cross-sectional region of the virtual implant abutment and thus may generate the optimal occlusal adjustment region.

In the case where the temporary crown and the antagonist tooth are aligned in such a manner as to be engaged with each other, the optimal occlusal adjustment region generation unit 6000 may select a plurality of polygons belonging to the three dimensional model for the antagonist tooth corresponding to at least two regions where the surface of the temporary crown and the surface of the antagonist tooth are brought into contact with each other, may select a preset number or greater number of a plurality of polygons adjacent to the plurality of polygons selected and set a candidate occlusal adjustment polygon group on a per-region basis, may select at least one polygon on a per-candidate occlusal adjustment polygon group basis in such a manner that the direction of the composite vector based on the orthogonal vectors orthogonal to the respective surfaces of a plurality of polygons belonging to each of the different candidate occlusal adjustment polygon groups is toward the lower cross-sectional region of the virtual implant abutment, and may generate an optimal occlusal adjustment region made up of a combination of polygons selected on a per-candidate occlusal adjustment polygon group basis.

The image generation unit 7000 may generate an optimal occlusal adjustment region image for displaying the optimal occlusal adjustment region on the three-dimensional model for the antagonist tooth and an antagonist tooth region image for displaying the three-dimensional model for the antagonist tooth region.

The display unit 8000 may display the optimal occlusal adjustment region image on the antagonist tooth region image in an overlapping manner.

A user, such as a dental technician, may check the optimal occlusal adjustment region derived from the primarily arranged temporary crown and may design a shape of the implant capable of minimizing a rotational moment to be applied to an implant without separate stress analysis by changing a shape of the temporary crown in such a manner that a region where the temporary crown and the antagonist tooth are adjusted occlusally corresponds to the optimal occlusal adjustment region.

The method of recommending an optimal occlusal adjustment region for an implant by utilizing a computer according to the third embodiment not covered by the present invention may be implemented with a computer program. The method of recommending an optimal occlusal adjustment region for an implant by utilizing a computer may be implemented by being stored in a computer-readable medium and being read by a computer.

The method of recommending an optimal occlusal adjustment region for an implant by utilizing a computer may be performed by the apparatus 1 for recommending an optimal occlusal adjustment region for an implant by utilizing a computer according to the second embodiment not covered by the present invention.

In addition, data may be transmitted and received through wired or wireless communication between apparatuses that are used while performing the method of recommending an optimal occlusal adjustment region for an implant by utilizing a computer according to the second embodiment of the present invention.

For example, wireless communication standards, such as Wireless LAN (WLAN), Wi-Fi, Wibro, Wimax, and High Speed Downlink Packet Access, may be used. However, the apparatuses are not limited to these wireless communication standards and may use wired communication standards, such as Universal Serial Bus, Ethernet, xDSL (ADSL or VDSL), Hybrid Fiber Coaxial Cable (HFC), Fiber to the Curb (FTTC), and Fiber to the Home (FTTH), depending on how a system is realized. In addition, short-distance communication standards, such as Bluetooth, Radio Frequency Identification (RFID), Infrared Data Association (IrDA), Ultra-Wideband (UWB), ZigBee, and Near Field Communication (NFC), may be used.

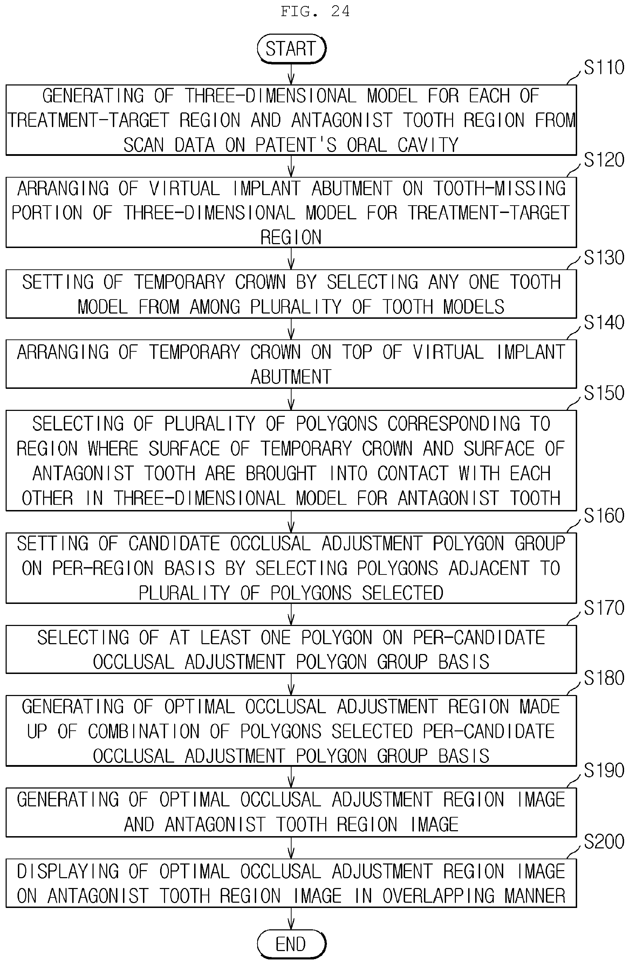

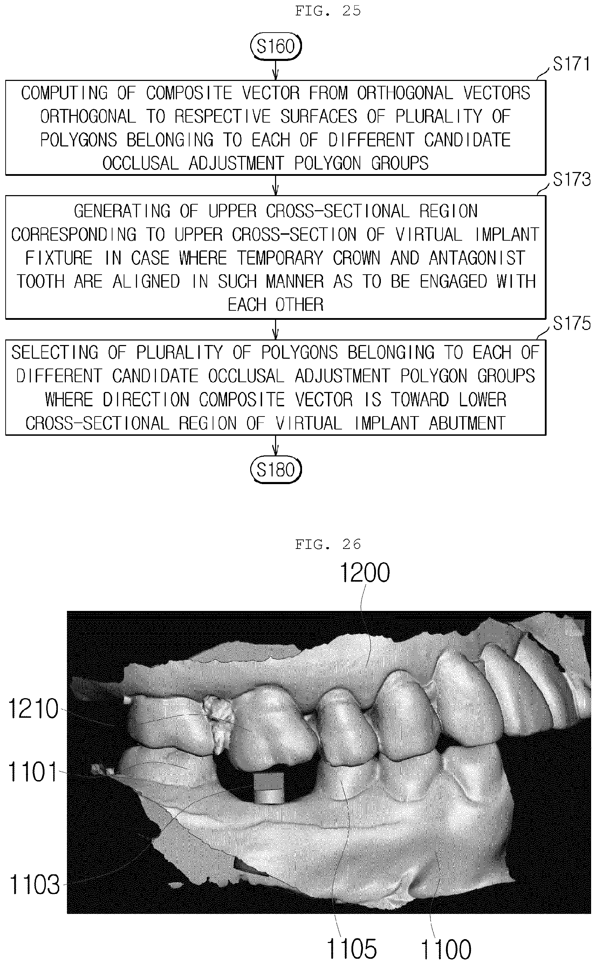

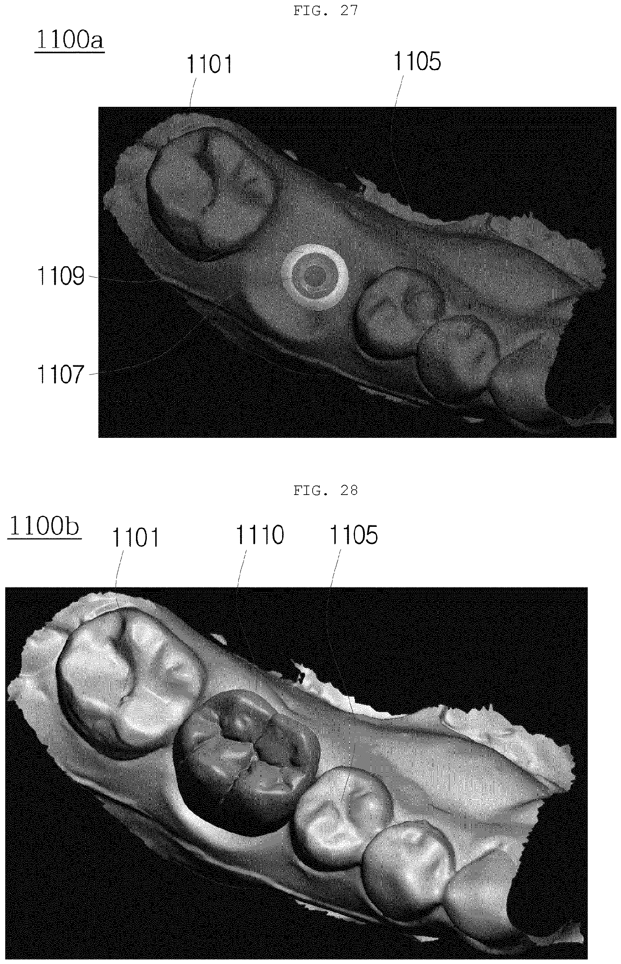

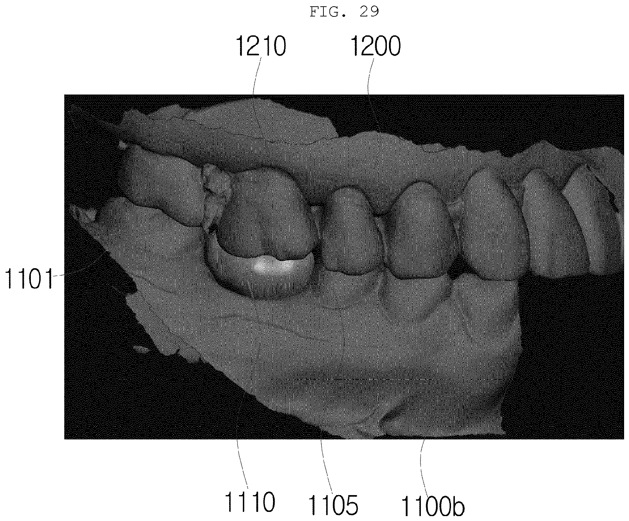

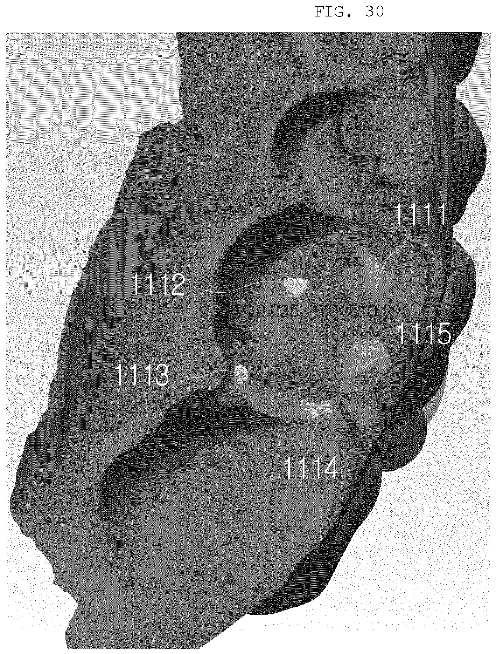

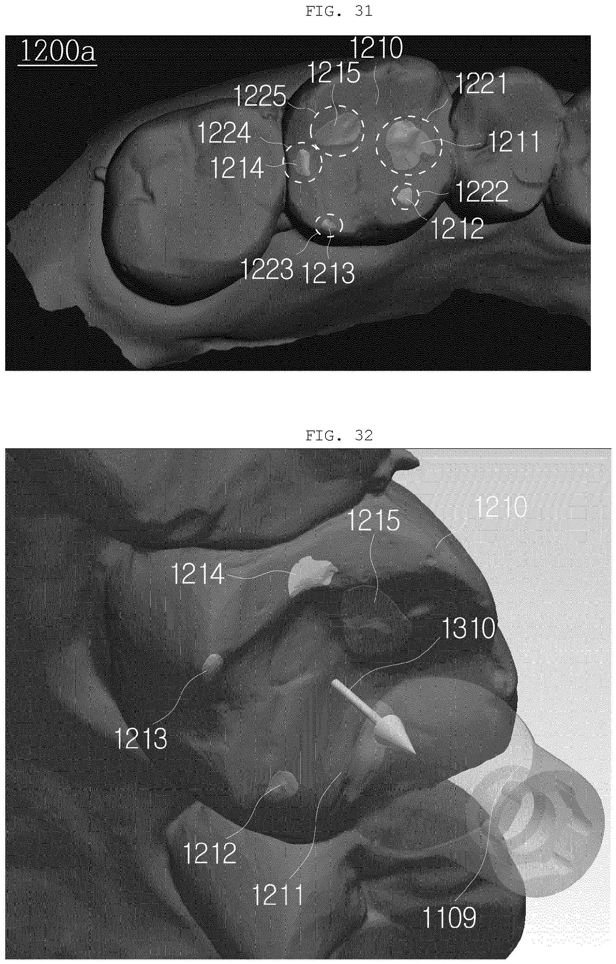

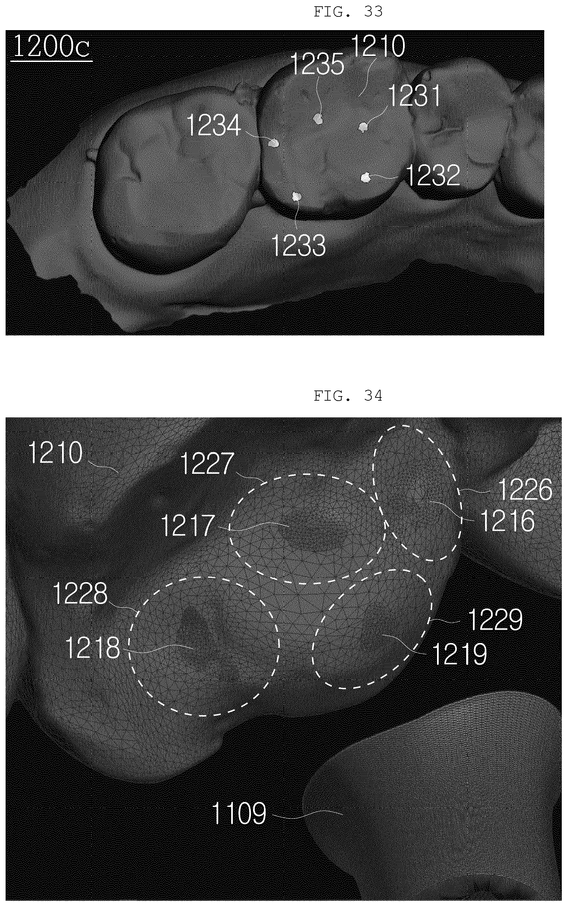

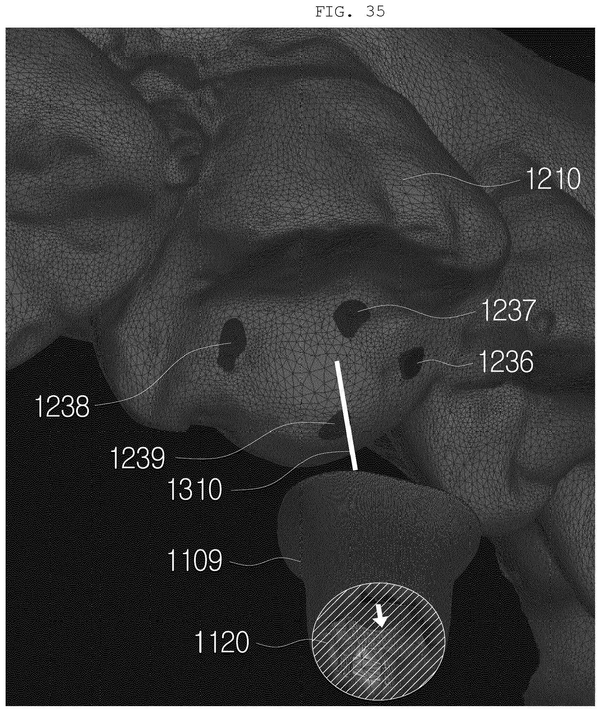

FIG. 24 is a block diagram illustrating the method of recommending an optimal occlusal adjustment region for an implant by utilizing a computer according to the third embodiment not covered by the present invention. FIG. 25 is a flowchart for selecting at least one polygon on a per-candidate occlusal adjustment polygon group basis in FIG. 24. FIG. 26 is an exemplary screen view illustrating the three-dimensional model generated for each of the treatment-target region and the antagonist tooth region according to the third embodiment of the present invention. FIG. 27 is an exemplary screen view illustrating the three-dimensional model for the treatment-target region where the virtual implant abutment is arranged according to the third embodiment not covered by the present invention. FIG. 28 is an exemplary screen view illustrating the three-dimensional model for the treatment-target region where the temporary crown is arranged according to the third embodiment not covered by the present invention. FIG. 29 is an exemplary screen view illustrating that the three-dimensional model for the treatment-target region where the temporary crown is arranged and the three-dimensional model for the antagonist tooth region are aligned in such a manner as to be engaged with each other according to the third embodiment not covered by the present invention. FIG. 30 is an exemplary screen view illustrating a region where the surface of the temporary crown and the surface of the antagonist tooth are brought into contact with each other according to the third embodiment not covered by the present invention. FIG. 31 is an exemplary screen view illustrating the candidate occlusal adjustment polygon group according to the third embodiment not covered by the present invention. FIG. 32 is an exemplary screen view illustrating the composite vector according to the third embodiment not covered by the present invention. FIG. 33 is an exemplary screen view illustrating the optimal occlusal adjustment region according to the third embodiment not covered by the present invention. FIG. 34 is an exemplary screen view illustrating a plurality of polygons selected in a manner that corresponds to a region where the temporary crown and the antagonist tooth are engaged with each other and the candidate occlusal adjustment polygon group according to the third embodiment not covered by the present invention. FIG. 35 is an exemplary screen view illustrating the optimal occlusal adjustment region made up of a combination of a plurality of polygons according to the third embodiment not covered by the present invention. The method of recommending an optimal occlusal adjustment region for an implant by utilizing a computer will be described in detail with reference to FIGS. 24 to 35.

With reference to FIG. 24, the method of recommending an optimal occlusal adjustment region for an implant by utilizing a computer according to the third embodiment not covered by the present invention may include Step S110 of generating a three-dimensional model; Step S120 of arranging a virtual implant abutment; Step S130 of setting a temporary crown; Step S130 of arranging the temporary crown on the top of the virtual implant abutment; Step S150 of selecting a plurality of polygons corresponding to a region where a surface of the temporary crown and a surface of an antagonist tooth are brought into contact with each other; Step S160 of setting a candidate occlusal adjustment polygon group on a per-region basis; Step S170 of selecting at least one polygon on a per-candidate occlusal adjustment polygon group basis; Step S180 of generating an optimal occlusal adjustment region; Step S190 of generating an image for displaying the optimal occlusal adjustment region on the top of the antagonist tooth; and Step S200 of displaying the generated image.

First, a three-dimensional model 1100 for the treatment-target region including a treatment-target region including a scan body 1103 and a three-dimensional model 1200 for an antagonist tooth region facing the treatment-target region are generated from scan data generated by scanning a patient's oral cavity in which the scan body 1200 is fastened to the tooth-missing portion, using an intraoral scanner (S1100).

During a three-dimensional scan process, an implant fixture implanted inside the missing-tooth portion is not scanned. Therefore, in order to specify a position of the implant fixture, the scan body 1200 is fastened to the top of the implant fixture implanted into the patient's gum, and the scan data generated by scanning the patient's oral cavity where the scan body 1200 is fastened are used.

The treatment-target region may include a region that is made up of a region where an implant operation is performed and a region where an adjacent tooth is positioned adjacent to the region where the implant operation is performed. The antagonist tooth region may be a region including a teeth arrangement facing the treatment-target region.

At this point, each of the three-dimensional models 1100 for the treatment-target region and the three-dimensional models 1200 for the antagonist tooth region may be made up of a combination of a plurality of polygons.

Subsequently, a virtual implant abutment 1109 is arranged inside the missing-tooth portion of the three-dimensional model 1100 for the treatment-target region (S120).

In Step of arranging the virtual implant abutment 1109, the virtual implant abutment 1109 may be arranged on the basis of a position of the scan body 1103 of the three-dimensional model 1100 for the treatment-target region (S120).

More specifically, a preset virtual implant fixture 1107 may be arranged according to the position of the scan body 1103 of the three-dimensional model 1100 for the treatment-target region, and the virtual implant abutment 1109 may be arranged on the top of the arranged virtual implant fixture 1107 in such a manner that a lower end of the virtual implant abutment 1109 is engaged with an upper end of the virtual implant fixture 1107 (S120).

After the virtual implant abutment 1109 is arranged, any one tooth model is selected from among a plurality of tooth models as prestored three-dimensional models and the selected one tooth model is set to be a temporary crown (S130).

The plurality of tooth models may be stored in such a manner as to correspond to a plurality of tooth positions 11 to 18, 21 to 28, 31 to 38, and 41 to 48, respectively.

A three-dimensional model for a tooth at the corresponding tooth position may be generated from the scan data generated by scanning the patient's oral cavity in the past, and the generated three-dimensional model for the corresponding tooth position may be stored.

The plurality of tooth models may be stored on a tooth library in a computer. Three-dimensional tooth models on a per-tooth shape basis may be categorized according to each tooth position, and the resulting three-dimensional tooth models may be stored in the tooth library.

Any one tooth model corresponding to a position of the tooth-missing portion may be selected from among a plurality of tooth models as prestored three-dimensional modes, according to a position of the virtual implant abutment 1109 on the three-dimensional model 1100 for the treatment-target region, that is, according to a position of the tooth-missing portion, and the selected one tooth model may be set to be the temporary crown (S130).

Subsequently, a size of a temporary crown 1110 is adjusted according to respective shapes of adjacent teeth 1101 and 1105 adjacent to the tooth-missing portion in the three-dimensional model 1100 for the treatment-target region and to a shape of an antagonist tooth 1210 corresponding to the tooth-missing portion in the three-dimensional model 1200 for the antagonist tooth region, and thus the temporary crown 1110 whose size is adjusted is arranged on the top of the virtual implant abutment 1109 (S140).

More specifically, a width or length of the temporary crown 1110 may be increased or decreased in such a manner that a distance between the temporary crown 1110 and each of the adjacent teeth 1101 and 1105 is equal to or smaller than a preset distance (for example, 0.01 mm) by computing a width between the adjacent teeth 1101 and 1105. Alternatively, a height of the temporary crown 1110 may be increased or decreased in such a manner that the height of the temporary crown 1110 is the same as respective heights of the adjacent teeth 1101 and 1105 by computing the respective heights of the adjacent teeth 1101 and 1105. In this manner, the size of the temporary crown 1110 may be adjusted and thus the temporary crown 1110 whose size is adjusted may be arranged on the top of the virtual implant abutment 1109 (S140).

After the temporary crown 1110 is arranged on the top of the virtual implant abutment 1109, in a case where the temporary crown 1110 and the antagonist tooth 1210 are aligned in such a manner as to be engaged with each other, a plurality of polygons 1211, 1212, 1213, 1214, and 1215 belonging to the three-dimensional model 1200 for the antagonist tooth 1210 corresponding to at least two regions, among regions 1111, 1112, 1113, 1114, and 1115 where a surface of the temporary 1110 and a surface of the antagonist tooth 1210 are brought into contact with each other are selected (S150).

In a state where a three-dimensional model 1100b for the treatment-target region where the temporary crown 1110 is arranged and the three-dimensional model 1200 for the antagonist tooth region are engaged with each other, a plurality of polygons 1211, 1212, 1213, 1214, 1215, 1216, 1217, 1218, and 1219 belong to a surface of the antagonist tooth 1210 that remains as a result of subtracting a volume of the temporary crown 1110 from a volume of the antagonist tooth 1210 are selected. Accordingly, a plurality of polygons 1211, 1212, 1213, 1214, and 1215 belonging to the three-dimensional model 1200 for the antagonist tooth region 1210 corresponding to at least two regions, among the regions 1111, 1112, 1113, 1114, and 1115 where the surface of the temporary 1110 and the surface of the antagonist tooth 1210 are brought into contact with each other may be selected (S150).

A preset number or greater number of a plurality of polygons adjacent to the plurality of polygons 1211, 1212, 1213, 1214, 1215, 1216, 1217, 1218, and 1219 that are selected in such a manner as to correspond to a region where the surface of the temporary crown 1110 and the surface of the antagonist tooth 1210 are brought into contact with each other in the three-dimensional model 1200 for the antagonist tooth 1210 are selected, and candidate occlusal adjustment polygon groups 1221, 1222, 1223, 1224, 1225, 1226, 1227, 1228, and 1229 are set on a per-region basis (S160).

Respective orthogonal vectors with respect to the plurality of selected polygons 1211, 1212, 1213, 1214, 1215, 1216, 1217, 1218, and 1219 are synthesized between the step of selecting the plurality of polygons 1211, 1212, 1213, 1214, 1215, 1216, 1217, 1218, and 1219 belonging to the three-dimensional model 1200 for the antagonist tooth corresponding to at least two regions where the surface of the temporary crown 1110 and the surface of the antagonist tooth 1210 are brought into contact with each other and the step of setting the candidate occlusal adjustment polygon groups 1221, 1222, 1223, 1224, 1225, 1226, 1227, 1228, and 1229. Accordingly, an initial composite vector in which external force vectors applied by the antagonist tooth 1210 to the temporary crown 1110 when the temporary crown 1110 and the antagonist tooth 1210 are engaged with each other may be derived, and the three-dimensional model 1100b for the treatment-target region where the temporary crown 1110 is arranged and the initial composite vector may be output on a screen.

The user, such as a dental technician, can check the three-dimensional model 1100b for the treatment-target region where the temporary crown 1110 is arranged and the initial composite vector and can adjust the shape of the temporary crown 1110.

In a case where the user adjusts the shape of the temporary crown 1110, when the temporary crown 1110 whose shape is adjusted and the antagonist tooth 1210 are aligned in such a manner as to be engaged with each other, a plurality of polygons belonging to the three-dimensional model 1200 for the antagonist tooth 1210 corresponding to at least two regions where the surface of the temporary crown 1110 and the surface of the antagonist tooth 1210 are brought into contact with each other may be selected, a preset or greater number of a plurality of polygons adjacent to the plurality of polygons selected may be selected, and the candidate occlusal adjustment polygon group may be set on a per-region basis.

After the step of setting the candidate occlusal adjustment polygon group, at least one polygon is selected for each of the candidate occlusal adjustment polygon groups 1221, 1222, 1223, 1224, 1225, 1226, 1227, 1228, and 1229 where the direction of the composite vector based on the orthogonal vectors orthogonal to respective surfaces of the plurality of polygons belonging to each of the candidate occlusal adjustment polygon groups 1221, 1222, 1223, 1224, 1225, 1226, 1227, 1228, and 1229 that are set, is toward a lower cross-sectional region 1120 of the virtual implant abutment 1109 (S170).

At this point, as a result of this selection, the closer the vector composition is to the center of the lower cross-sectional region 1120 of the virtual implant abutment 1109, the more decreased the rotational moment applied to the implant. Accordingly, the implant can be prevented from being damaged by the rotational moment.

The step of selecting at least one polygon on a per-candidate occlusal adjustment polygon group basis may include: Step S171 of computing a composite vector resulting from synthesizing orthogonal vectors orthogonal to respective surface of a plurality of polygons belonging to each combination according to a combination of a plurality of polygons belonging to each of the different candidate occlusal adjustment polygon groups 1221, 1222, 1223, 1224, 1225, 1226, 1227, 1228, and 1229; Step of S173 of generating a lower cross-sectional region 1120 corresponding to a lower cross-section of a virtual implant abutment 1109 in a case where a temporary crown 1110 and an antagonist tooth 1210 are aligned in such a manner as to be engaged with each other; and Step S175 of selecting a plurality of polygons belonging to each of the different candidate occlusal adjustment polygon groups 1221, 1222, 1223, 1224, 1225, 1226, 1227, 1228, and 1229 where a composite vector calculated is toward a lower cross-sectional region 1120 of the virtual implant abutment 1109.

In the step of generating the lower cross-sectional region, the lower cross-sectional region 1120 corresponding to a region where the virtual implant abutment 1109 and the virtual implant fixture 1107 are brought into contact with each other may be generated.

In Step S175 of selecting a plurality of polygons, X-axis coordinate values of a plurality of polygons corresponding to each composite vector may be averaged, Y-axis coordinate values thereof may be averaged, and Z-axis coordinate values thereof may be averaged. Thus, the center point may be computed. A starting point of the composite vector may be set to be the center point. In a case where the direction of the composite vector whose center point is set as the starting point is toward the lower cross-sectional region 1120 of the virtual implant abutment 1109, a plurality of polygons corresponding to the composite vector may be selected (S175).

In a case where there are a plurality of combinations of a plurality of polygons that satisfy a condition that the direction of the composite vector is toward the lower cross-sectional region 1120 of the virtual implant abutment 1109, a combination of a plurality of polygons corresponding to a composite vector 1310 that is toward a direction closest to the center of the lower cross-sectional region 1120, among composite vectors according to each combination of polygons selected, may be selected(S175) .

That is, when a polygon serving as a basis of computing the composite vector varies in characteristics, the center depending on respective positions of specific polygons and the direction of the composite vector vary. When the direction of the composite vector varies, in the case where the antagonist tooth and the temporary crown are engaged with each other, stress applied to the implant fixture through the virtual implant abutment from the antagonist tooth varies.

In the method of and the apparatus for recommending an optimal occlusal adjustment region for an implant by utilizing a computer according to the third and second embodiments not covered by the present invention, according to the above-described process, a plurality of combinations of polygons where the direction of the composite vector in the center according to respective positions of the polygons is toward the center of a combination surface where the implant fixture and the virtual implant abutment are combined with each other are selected.

Parameters for a preset neural structure are predefined using the composite vector that results from synthesizing the orthogonal vectors orthogonal to respective surfaces of a plurality of polygons or the orthogonal vectors, and using guidance learning values corresponding to the composite vector that is toward the direction closest to the center of the lower cross-sectional region 1120 of the virtual implant abutment 1109. Accordingly, a plurality of polygons where the direction of the composite vector is toward the direction closest to the center of the lower cross-sectional region 1120 of the virtual implant abutment 1109 by applying the orthogonal vectors orthogonal to respective surfaces of polygons belonging to the candidate occlusal adjustment polygon group to a learning model that is pre-learned (S175).

In order to derive the composite vector representing the direction closest to the center of the lower cross-sectional region 1120 of the virtual implant abutment 1109, among a plurality of composite vectors, an adaptive grid search for searching for an optimal parameter by applying all derivable combinations derivable from a plurality of parameters, convex optimization for searching for a point having an optimal value with respect to a preset function, or the like may be performed.

Subsequently, the optimal occlusal adjustment regions 1231, 1232, 1233, 1234, 1235, 1236, 1237, 1238, and 1239 each of which is made up of combinations of polygons selected for each of the candidate occlusal adjustment polygon groups 1221, 1222, 1223, 1224, 1225, 1226, 1227, 1228, and 1229 are generated (S180).

At this point, the plurality of optimal occlusal adjustment regions 1231, 1232, 1233, 1234, 1235, 1236, 1237, 1238, and 1239) each of which is made up of combinations of polygons selected for each of the candidate occlusal adjustment polygon groups 1221, 1222, 1223, 1224, 1225, 1226, 1227, 1228, and 1229, and that depend on a preset number of optimal occlusal adjustment points (which, for example, ranges from three to eight) may generated (S180).

In the case where the temporary crown 1110 and the antagonist tooth 1210 are aligned in such a manner as to be aligned with each other, the plurality of polygons 1211, 1212, 1213, 1214, 1215, 1216, 1217, 1218, and 1219 belonging to the three-dimensional model 1200 for the antagonist tooth 1210 corresponding to at least two regions, among the regions 1111, 1112, 1113, 1114, and 1115 where the surface of the temporary crown 1110 and the surface of the antagonist tooth 1210 are brought into contact with each other are selected. In addition, according to the second and third embodiments of the present invention, the optimal occlusal adjustment regions 1231, 1232, 1233, 1234, 1235, 1236, 1237, 1238, and 1239 are generated using a plurality of candidate occlusal adjustment polygon groups based on the plurality of polygons selected.

More specifically, the optimal occlusal adjustment regions 1231, 1232, 1233, 1234, 1235, 1236, 1237, 1238, and 1239 where a direction of the composite vector 1310 based on the orthogonal vectors orthogonal to respective surfaces of a plurality of polygons selected for each of the candidate occlusal adjustment polygon groups is toward the center of the lower cross-sectional region 1120 of the virtual implant abutment 1109 are generated.

According to the embodiment not covered by the present invention, a plurality of polygons where the direction of the composite vector based on the orthogonal vector orthogonal to the polygon is toward an upper cross-sectional region of the virtual implant fixture are selected from among a plurality of polygons belonging to the candidate occlusal adjustment polygon groups generated on a basis of the region where the temporary crown and the antagonist tooth are engaged with each other. Thus, the occlusal adjustment point for analyzing stress against the crown on the implant and minimizing the stress can be automatically derived.

According to the second and third embodiments not covered by the present invention, the optimal occlusal adjustment regions 1231, 1232, 1233, 1234, 1235, 1236, 1237, 1238, and 1239 are generated on the basis of the temporary crown in accordance with the tooth model prestored according to the tooth position, and thus a crown in the shape of a natural tooth can be designed.

After the optimal occlusal adjustment regions 1231, 1232, 1233, 1234, 1235, 1236, 1237, 1238, and 1239 are generated, the optimal occlusal adjustment region image for displaying the optimal occlusal adjustment regions 1231, 1232, 1233, 1234, 1235, 1236, 1237, 1238, and 1239 on the three-dimensional model 1200 for the antagonist tooth 1210 and the antagonist tooth region for displaying the three-dimensional model 1200 for the antagonist tooth 1210 are generated (S190). The optimal occlusal adjustment image is displayed on the antagonist tooth region image in an overlapping manner (S200).

According to the embodiment not covered by the present invention, the optimal occlusal adjustment region where the direction of the external force vector applied from the antagonist tooth to the implant is toward the lower cross-sectional region of the virtual implant abutment, ideally, the center portion of the lower cross-sectional region is generated, and the generated optimal occlusal adjustment region is displayed on the antagonist tooth. Thus, the implant crown capable of minimizing the rotational moment to be applied to the implant can be designed without the need for expertise in stress analysis.

With the method of and the apparatus for recommending an optimal occlusal adjustment region for an implant by utilizing a computer according to the third and second embodiments not covered by the present invention, referring to the optimal occlusal adjustment region displayed on the antagonist tooth, the user, such as a dental technician, can design the temporary crown in such a manner that the region where the antagonist tooth and the temporary crown are engaged with each other corresponds to the optimal occlusal adjustment region, by partially raising or recessing the surface of the temporary crown. Thus, the user can design the shape of the crown on the implant capable of minimizing the stress to be applied to the implant without separate stress analysis.

It would be understandable by a person of ordinary skill in the art to which the present invention pertains that the present invention can be practiced in other specific forms without any modification to the present invention. Therefore, in every aspect, the embodiments described above should be understood as being exemplary and non-restrictive. The scope of the present invention is defined by the claims in light of the specification. All modifications or substitutions derived from the claims thereof should be interpreted as falling within the scope of the present invention.

### Industrial Applicability

The method of forming, by utilizing a computer, an implant shape for minimizing stress to be applied to an implant, and the method of and the apparatus of recommending an optimal occlusal adjustment region for an implant according to the present invention can be used in technical fields, such as virtual tooth stress analysis and implant design and manufacturing.

## Claims

1. A method of forming, by utilizing a computer, an implant shape for minimizing stress to be applied to an implant, the method comprising:
a tooth scan model generation step (S1) of scanning a treatment-target region and a teeth arrangement facing the treatment-target region for detection using an intraoral scanner, generating scan data, and generating a three-dimensional model for each of the treatment-target region and the teeth arrangement facing the treatment-target region on the basis of the generated scan data;
a scan file alignment step (S2) of aligning a three-dimensional model (10) for the treatment-target region and a three-dimensional model (20) for the teeth arrangement facing the treatment-target region in such a manner as to be engaged with each other, the model (10) and the model (20) being generated in the tooth scan model generation step (S1);
a virtual implant arrangement step (S3) of positioning a virtual implant (100) in a missing-tooth portion (11) of the three-dimensional model (10) for the treatment-target region, the model (10) being generated in the tooth scan model generation step (S1);
a first temporary crown arrangement step (S4) of automatically arranging an arbitrarily selected first temporary crown (200a) on the virtual implant (100) arranged in the virtual implant arrangement step (S3) and connecting the arbitrarily selected first temporary crown (200a) to a connection portion (110) of the virtual implant (100);
a second temporary crown substitution step (S5) of substituting a second temporary crown (200b) for the first temporary crown (200a) arranged in the first temporary crown arrangement step (S4) on the basis of a relationship between each of the adjacent teeth (12) and (13) adjacent to the missing-tooth portion (11) and an antagonist tooth (21) corresponding to the missing-tooth portion (11);
an occlusal adjustment point extraction step (S6) of extracting an occlusal adjustment point (201b) at which the second temporary crown (200b) is engaged with a missing-tooth-engaged antagonist tooth (21), after the second temporary crown substitution step (S5);
a second temporary crown occlusal adjustment region derivation step (S7) of deriving an occlusal adjustment region (202b) of the second temporary crown (200b) that is a surface remaining after subtracting a volume of the antagonist tooth (21) from a volume of the second temporary crown (200b), in a state where the three-dimensional model (10) for the treatment-target region including the second temporary crown (200b) and the three-dimensional model (20) for the teeth arrangement facing the treatment-target region including the antagonist tooth (21) are engaged with each other, after the occlusal adjustment point extraction step (S6);
a vector derivation step (S8) of deriving a single vector in which external force vectors applied by the antagonist tooth (21) to all local portions in the occlusal adjustment region (202b) of the second temporary crown (200b) derived in the second temporary crown occlusal adjustment region derivation step (S7) are equivalent to each other; and
a crown shape determination step (S9) of determining a shape of the second temporary crown (200b) in a case where a value of the single vector derived in the vector derivation step (S8) is such that an angle R with respect to an axis of the virtual implant (100) is equal to or smaller than a predetermined angle.

2. The method of claim 1,
wherein in a case where the value of the single vector derived in the vector derivation step (S8) is such that the angle R with respect to the axis of the virtual implant (100) falls outside a range from 0 degrees to 10 degrees, the occlusal adjustment point (201b) of the second temporary crown (200b) is redetermined on the basis of an input from a user, and the steps (S6) and (S7) are reiterated.

3. The method of claim 1,
wherein a crown shape of the first temporary crown (200a) in the first temporary crown arrangement step (S4) is determined using positional information of a missing tooth, and the second temporary crown (200b) in the second temporary crown substitution step (S5) is provided in such a manner that a size of the second temporary crown (200b) is adjusted considering a relationship between each of the adjacent teeth (12) and (13) positioned to the left and right, respectively, of the missing tooth and the antagonist tooth (21) engaged with the missing tooth.

4. The method of claim 1,
wherein three to eight occlusal adjustment points (201b) in the occlusal adjustment point extraction step (S6) are extracted.

## Patentansprüche

1. Verfahren zur Bildung einer Implantatform unter Verwendung eines Computers zur Minimierung der auf ein Implantat einwirkenden Belastung, wobei das Verfahren umfasst:
einen Schritt (S1) zum Erzeugen eines Zahnscanmodells, bei dem ein Behandlungszielbereich und eine dem Behandlungszielbereich gegenüberliegende Zahnanordnung unter Verwendung eines Intraoralscanners zur Erfassung gescannt werden, Scandaten erzeugt werden und ein dreidimensionales Modell für jeden Behandlungszielbereich und die Zahnanordnung, die dem Behandlungszielbereich gegenüberliegt, basierend auf den erzeugten Scandaten erzeugt wird;
einen Schritt (S2) zum Ausrichten von Scandateien, bei dem ein dreidimensionales Modell (10) für den Behandlungszielbereich und ein dreidimensionales Modell (20) für die dem Behandlungszielbereich gegenüberliegende Zahnanordnung so ausgerichtet werden, dass sie miteinander in Eingriff stehen, wobei das Modell (10) und das Modell (20) in dem Schritt (S1) der Erzeugung eines Zahnscanmodells erzeugt werden;
einen Schritt (S3) der Anordnung eines virtuellen Implantats, bei dem ein virtuelles Implantat (100) in einem Abschnitt (11), in dem ein Zahn fehlt, des dreidimensionalen Modells (100) für den Behandlungszielbereich positioniert wird, wobei das Modell (10) in dem Schritt (S1) der Erzeugung eines Zahnscanmodells erzeugt wird;
einen Schritt (S4) zum Anordnen einer ersten provisorischen Krone, bei dem eine willkürlich ausgewählte erste provisorische Krone (200a) automatisch auf dem virtuellen Implantat (100) angeordnet wird, das im Schritt (S3) der Anordnung eines virtuellen Implantats angeordnet wurde, und die willkürlich ausgewählte erste provisorische Krone (200a) mit einem Verbindungsabschnitt (110) des virtuellen Implantats (100) verbunden wird;
einen Schritt (S5) zum Ersetzen durch eine zweite provisorische Krone, bei dem die erste provisorische Krone (200a), die im Schritt (S4) der Anordnung einer ersten provisorischen Krone angeordnet wurde, basierend auf einer Beziehung zwischen jedem der benachbarten Zähne (12) und (13), die an den Abschnitt (11), in dem ein Zahn fehlt, angrenzen, und einem Antagonistenzahn (21), der dem Abschnitt (11), in dem ein Zahn fehlt, entspricht, durch eine zweite provisorische Krone (200b) ersetzt wird;
einen Schritt (S6) der Extraktion eines okklusalen Einstellpunkts, bei dem der okklusale Einstellpunkt (201b) extrahiert wird, an dem die zweite provisorische Krone (200b) mit einem Antagonistenzahn (21) in Eingriff steht, der mit einem fehlenden Zahn in Eingriff steht, nach dem Schritt (S5) der Ersetzung durch eine zweite provisorische Krone;
einen Schritt (S7) zum Ableiten eines okklusalen Einstellbereichs der zweiten provisorischen Krone, bei dem ein okklusaler Einstellbereich (202b) der zweiten provisorischen Krone (200b) abgeleitet wird, der eine Fläche ist, die nach dem Subtrahieren eines Volumens des Antagonistenzahns (21) von einem Volumen der zweiten provisorischen Krone (200b) verbleibt, in einem Zustand, in dem das dreidimensionale Modell (10) für den Behandlungszielbereich einschließlich der zweiten temporären Krone (200b) und das dreidimensionale Modell (20) für die Zahnanordnung, die dem Behandlungszielbereich einschließlich des Antagonistenzahns (21) gegenüberliegt, miteinander in Eingriff stehen, nach dem Schritt (S6) der Extraktion eines okklusalen Einstellpunkts;
einen Schritt (S8) zum Ableiten eines Vektors, bei dem ein einzelner Vektor abgeleitet wird, in dem äußere Kraftvektoren, die durch den Antagonistenzahn (21) auf alle lokalen Abschnitte in dem okklusalen Einstellbereich (202b) der zweiten temporären Krone (200b) ausgeübt werden, die in dem Schritt (S7) der Ableitung eines okklusalen Einstellbereichs der zweiten provisorischen Krone abgeleitet wurden, einander gleichwertig sind; und
einen Schritt (S9) zum Bestimmen der Kronenform, bei dem die Form der zweiten provisorischen Krone (200b) in einem Fall bestimmt wird, in dem ein Wert des einzelnen Vektors, der in dem Schritt (S8) der Ableitung eines Vektors abgeleitet wird, so ist, dass ein Winkel R in Bezug auf eine Achse des virtuellen Implantats (100) gleich oder kleiner als ein vorbestimmter Winkel ist.

2. Verfahren nach Anspruch 1,
wobei in einem Fall, in dem der Wert des einzelnen Vektors, der in dem Schritt (S8) der Ableitung eines Vektors abgeleitet wird, so ist, dass der Winkel R in Bezug auf die Achse des virtuellen Implantats (100) außerhalb eines Bereichs von 0 Grad bis 10 Grad liegt, der okklusale Einstellpunkt (201b) der zweiten provisorischen Krone (200b) basierend auf einer Eingabe von einem Nutzer neu bestimmt wird und die Schritte (S6) und (S7) wiederholt werden.

3. Verfahren nach Anspruch 1,
wobei eine Kronenform der ersten temporären Krone (200a) in dem Schritt (S4) der Anordnung der ersten provisorischen Krone unter Verwendung der Positionsinformationen eines fehlenden Zahns bestimmt wird und die zweite provisorische Krone (200b) in dem Schritt (S5) der Ersetzung durch eine zweite provisorische Krone so bereitgestellt wird, dass eine Größe der zweiten provisorischen Krone (200b) unter Berücksichtigung einer Beziehung zwischen jedem der benachbarten Zähne (12) und (13), die links bzw. rechts von dem fehlenden Zahn positioniert sind, und dem Antagonistenzahn (21), der mit dem fehlenden Zahn in Eingriff steht, eingestellt wird.

4. Verfahren nach Anspruch 1, wobei
drei bis acht okklusale Einstellpunkte (201b) in dem Schritt (S6) der Extraktion eines okklusalen Einstellpunkts extrahiert werden.

## Revendications

1. Procédé de façonnage, au moyen d'un ordinateur, d'une forme d'implant visant à minimiser la contrainte à appliquer à un implant, ledit procédé comprenant :
une étape de génération de modèle scanné de dent (S1), où sont scannées une zone cible de traitement et la disposition des dents opposées à la zone cible de traitement pour une détection au moyen d'un scanner intraoral, de génération de données de scan, et de génération d'un modèle tridimensionnel par la zone cible de traitement et pour la disposition des dents opposées à la zone cible de traitement sur la base des données de scan générées ;
une étape d'alignement de fichiers de numérisation (S2), où sont alignés un modèle tridimensionnel (10) pour la zone cible de traitement et un modèle tridimensionnel (20) pour la disposition des dents opposées à la zone cible de traitement, de manière à les accoupler l'un à l'autre, le modèle (10) et le modèle (20) étant générés lors de l'étape de génération de modèle scanné de dent (S1) ;
une étape de disposition d'implant virtuel (S3), où un implant virtuel (100) est positionné dans une partie de dent manquante (11) du modèle tridimensionnel (10) pour la zone cible de traitement, le modèle (10) étant généré lors de l'étape de génération de modèle scanné de dent (S1) ;
une étape de disposition de première couronne temporaire (S4), où est disposée automatiquement une première couronne temporaire (200a) sélectionnée arbitrairement sur l'implant virtuel (100) disposé lors de l'étape de disposition d'implant virtuel (S3), et où la première couronne temporaire (200a) sélectionnée arbitrairement est raccordée à une partie de connexion (110) de l'implant virtuel (100) ;
une étape de substitution d'une deuxième couronne temporaire (S5), où une deuxième couronne temporaire (200b) est substituée pour la première couronne temporaire (200a) disposée lors de l'étape de disposition de première couronne temporaire (S4) sur la base d'une relation entre chacune des dents (12) et (13) adjacentes à la partie de dent manquante (11) et une dent antagoniste (21) correspondant à la partie de dent manquante (11) ;
une étape d'extraction de point d'ajustement occlusal (S6), où est extrait un point d'ajustement occlusal (201b) où la deuxième couronne temporaire (200b) est accouplée à une dent antagoniste accouplée à une dent manquante (21), après l'étape de substitution d'une deuxième couronne temporaire (S5) ;
une étape de déduction de zone d'ajustement occlusal de deuxième couronne temporaire (S7), où est déduite une zone d'ajustement occlusal (202b) de la deuxième couronne temporaire (200b), laquelle est une surface restant après soustraction d'un volume de la dent antagoniste (21) d'un volume de la deuxième couronne temporaire (200b), dans un état où le modèle tridimensionnel (10) pour la zone cible de traitement comprenant la deuxième couronne temporaire (200b) et le modèle tridimensionnel (20) pour la disposition des dents opposées à la zone cible de traitement comprenant la dent antagoniste (21) sont accouplés l'un à l'autre, après l'étape d'extraction de point d'ajustement occlusal (S6) ;
une étape de déduction de vecteur (SB), où est déduit un vecteur unique où des vecteurs de force externe, appliqués par la dent antagoniste (21) à toutes les parties locales dans la zone d'ajustement occlusal (202b) de la deuxième couronne temporaire (200b) déduite lors de l'étape de déduction de zone d'ajustement occlusal de deuxième couronne temporaire (S7), sont équivalents entre eux ; et
une étape de détermination de forme de couronne (S9), où est de déterminée une forme de la deuxième couronne temporaire (200b) dans le cas où une valeur du vecteur unique déduit lors de l'étape de déduction de vecteur (SB) est telle qu'un angle R par rapport à un axe de l'implant virtuel (100) est égal ou inférieur à un angle prédéterminé.

2. Procédé selon la revendication 1,
où, dans le cas où la valeur du vecteur unique déduit lors de l'étape de déduction de vecteur (SB) est telle que l'angle R par rapport à l'axe de l'implant virtuel (100) est extérieure à une plage de 0 à 10 degrés, le point d'ajustement occlusal (201b) de la deuxième couronne temporaire (200b) est redéterminé sur la base d'une entrée d'un utilisateur, et les étapes (S6) et (S7) sont répétées.

3. Procédé selon la revendication 1,
où une forme de couronne de la première couronne temporaire (200a) lors de l'étape de disposition de première couronne temporaire (S4) est déterminée au moyen d'informations de position d'une dent manquante, et où la deuxième couronne temporaire (200b) lors de l'étape de substitution d'une deuxième couronne temporaire (S5) est prévue de telle manière qu'une grandeur size de la deuxième couronne temporaire (200b) est ajustée en considération d'une relation entre chacune des dents adjacentes (12) et (13) positionnées à gauche et à droite, respectivement, de la dent manquante, et la dent antagoniste (21) accouplée à la dent manquante.

4. Procédé selon la revendication 1,
où de trois à huit points d'ajustement occlusaux (201b) sont extraits lors de l'étape d'extraction de point d'ajustement occlusal (S6).
